# EUROPEAN PATENT APPLICATION

(11) **EP 4 316 461 A1**
(43) Date of publication of application: **07.02.2024**
(21) Application number: 22188938.9
(22) Date of filing: 05.08.2022
(51) Int. Cl.: A61K 8/73, A61K 8/9789, A01N 43/16, C11D 3/48, A23L 3/3544, A61K 36/36, A61K 36/77, A61Q 5/00, A61Q 5/02, A61Q 5/06, A61Q 5/12, A61Q 9/02, A61Q 9/04, A61Q 15/00, A61Q 17/00, A61Q 17/04, A61Q 19/00, A61Q 19/08, A61Q 19/10, C11D 3/382, A61K 31/704

(54) **A PRESERVATIVE COMPOSITION**

(71) Applicant: BRAIN Biotech AG, 64673 Zwingenberg (DE); Analyticon Discovery GmbH, 14473 Potsdam (DE)
(72) Inventor: KATZER, Werner, 12207 Berlin (DE); Siems, Karsten, 14552 Michendorf (DE); TIFFERT, Yvonne, 68309 Mannheim (DE)
(74) Representative: Fabry, Bernd

(57) **Abstract**

Suggested is a preservative composition comprising or consisting of hederagenin glycosides, said hederagenin glycosides being selected from at least one monodesmosidic hederagenin-3-glycoside according to formula (I), A, B and C.

## Description

### AREA OF INVENTION

The present invention refers to the area of Hederagenin glycosides and concerns new and effective preservatives for cosmetic, detergent and food preparations of natural origin.

The rise of resistant microbes illustrates the need for further compounds able to prevent microbial infections and contaminations, while showing low toxicity in humans. Some triterpene glycosides have demonstrated anti-microbial properties. It is commonly observed in microorganisms that treatment with membrane disruptors can lead to cellular apoptosis. Therefore, triterpene glycosides show great potential for anti-microbial applications due to their high biological activity against microbes with low observed toxicity.

Triterpene glycosides are particularly useful as preservatives for foodstuff but also for other products of daily life, e.g. pet food and cosmetics. The high water and often high sugar content of food, pet food or cosmetic preparations, for instance, make them susceptible to contamination with microorganism. Many products of daily life that are not chemically or physically treated will spoil within a short period of time. Preservation methods such as pasteurization or addition of chemical preservatives such as sorbic or benzoic acid can have a profound impact on the sensory properties of the product. However, sensory changes of products upon preservation are undesirable as customers value natural flavor. Additionally, there is a need for alternatives to synthetically produced conservatives due to the high environmental cost of precursor compounds used for industrial production. Triterpene glycosides represent a suitable alternative to widely used preservation approaches due to their anti-microbial properties, low toxicity and more eco-friendly production.

### RELEVANT PRIOR ART

WO 2013 037818 A1 (WACKER) discloses ustilagic acid and its esters for fighting specific germs such as Alicylcobacillus acidoterrestris, *Penicillium expansum, Gluconacetobacter liquefociens, Lactobacillus plantarum, Zygosaccaromyces rouxii, Aspergillus brasiliensis, Brettanomyces naardanensis,* and *Weissella confusa* in non-alcoholic beverages. Ustilagic acids are glycosides with a long chain fatty acid and an attached disaccharide. Tamura et al. [Natural Meclicines 55 (1) ,11-16 (2001] found that the sapindoside mixture and the monodesmosidic saponin extracted from it had obvious inhibitory effect on dermatophytes *(Candida albicans, Trichophyton rubrum, Trichophyton mentagrophytes, Epidermophyte and Microsporum sp*.) and moderate activity on gram positive bacteria (*S. aureus, S. epidermidis, S. mutans, B. subtilis*), but no activity against gram negative bacteria (*E*. *coli, P. aeruginosa, Alcaligenes faecali, P. vulgaris*) and fungi (*A. niger, P. citrinum*). Synergistic antibacterial activity of Sapindoside A and Sapindoside B against *Micrococcus luteus* was described by Wei et al. [Food Control Volume 130, 2021, 108337].

### OBJECT OF THE INVENTION

Due to increasing resistance of specific microbes, particularly of germs like
- *Actinomyces viscosus*
- *Alicyclobacillus acidoterrestris*
- *Alternaria alternata*
- *Aspergillus brasiliensis*
- *Bacillus cereus*
- *Bacillus licheniformis*
- *Botrytis cinerea*
- *Brettanomyces naardenensis*
- *Byssochlamys fulva*
- *Campylobacter jejuni*
- *Candida albicans*
- *Candida parapsilosis*
- *Cladosporium cladosporioides*
- *Clostridium perfringens*
- *Clostridium sporogenes*
- *Corynebacterium xerosis*
- *Cutibacterium acnes*
- *Enterobacter cloacae*
- *Enterococcus faecalis*
- *Fusobacterium nucleatum subsp. vincentii*
- *Gluconacetobacter liquefaciens*
- *Lactobacillus paracasei ssp. paracasei*
- *Lactobacillus plantarum ssp. plantarum*
- *Leuconostoc lactis*
- *Leuconostoc mesenteroides ssp. mesenteroides*
- *Listeria monocytogenes*
- *Malassezia furfur*
- *Pasteurella multocida ssp. multocida*
- *Penicillium expansum*
- *Phanerochaete chrysosporium*
- *Porphyromonas gingivalis*
- *Pseudomonas aeruginosa*
- *Pseudomonas fluorescens*
- *Saccharomyces cerevisiae*
- *Salmonella enterica ssp. enterica Ser. typhimurium*
- *Staphylococcus aureus*
- *Streptococcus mutans*
- *Yarrowia lipolytica*
- *Zygosaccharomyces bailii*
- *Zygosaccharomyces rouxii.*
which are found in many products of daily life particularly in cosmetic, detergent and food preparations, there is demand for new and highly effective preservatives, particularly obtained from natural sources and capable of fighting these microbes, preferably at a very low application level.

### BRIEF DESCRIPTION OF THE INVENTION

A first object of the present invention refers to a preservative composition comprising or consisting of triterpene glycosides, said glycosides being selected from at least monodesmosidic hederagenin-3-glycoside according to formula (I) 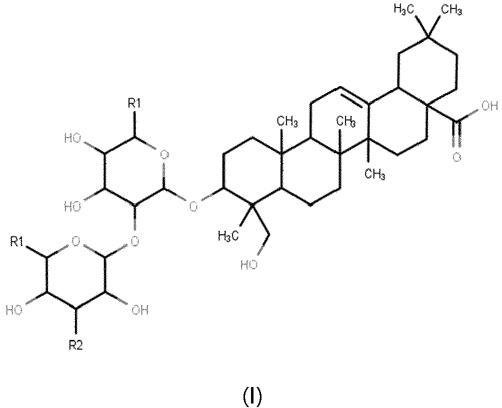 wherein
R1 stands for H, CH3, CH2OH or COOH,
R2 stands for one sugar selected from the group Glu, Gal, Rha, Ara, Xyl, Api, GIcA and mixtures thereof.

Surprisingly it has been found that the hederagenin-3-glycosides according to the present invention are active against a broad spectrum of microorganisms, including bacteria, fungi and yeasts, particularly against
- *Zygosaccharomyces sp.*
- *Actinomyces viscosus*
- *Alicyclobacillus acidoterrestris*
- *Alternaria alternata*
- *Aspergillus brasiliensis*
- *Bacillus cereus*
- *Bacillus licheniformis*
- *Botrytis cinerea*
- *Brettanomyces naardenensis*
- *Byssochlamys fulva*
- *Campylobacter jejuni*
- *Candida albicans*
- *Candida parapsilosis*
- *Cladosporium cladosporioides*
- *Clostridium perfringens*
- *Clostridium sporogenes*
- *Corynebacterium xerosis*
- *Cutibacterium acnes*
- *Enterobacter cloacae*
- *Enterococcus faecalis*
- *Fusobacterium nucleatum subsp. vincentii*
- *Gluconacetobacter liquefaciens*
- *Lactobacillus paracasei ssp. paracasei*
- *Lactobacillus plantarum ssp. plantarum*
- *Leuconostoc lactis*
- *Leuconostoc mesenteroides ssp. mesenteroides*
- *Listeria monocytogenes*
- *Malassezia furfur*
- *Pasteurella multocida ssp. multocida*
- *Penicillium expansum*
- *Phanerochaete chrysosporium*
- *Porphyromonas gingivalis*
- *Pseudomonas aeruginosa*
- *Pseudomonas fluorescens*
- *Saccharomyces cerevisiae*
- *Salmonella enterica ssp. enterica Ser. typhimurium*
- *Staphylococcus aureus*
- *Streptococcus mutans*
- *Yarrowia lipolytica*
- *Zygosaccharomyces bailii*
- *Zygosaccharomyces rouxii.*
already at very low working levels. The products are obtained by extraction of natural sources preferably using alcohols or mixtures of alcohol and water.

The preservative composition of the present invention is preferably applied for food products excluding beverages.

### Monodesmosidic hederagenin-3-glycosides

Monodesmosidic hederagenin-3-glycosides are saponins comprised of a single linear glycoside chain of three monosaccharides chemically bound to C-3 of the aglycon Hederagenin (3,23-Dihydroxy-12-oleanen-28-oic acid) through a glycosidic bond. Preferably no esters are attached to the monosaccharides. Hederagenin is a pentacyclic triterpenoid which is a chemical constituent of the *Hedera helix* (common ivy) plant but can be found in multiple plant genera. Hederagenin and its related Hederagenin glycosides show anti-inflammatory and anti-cancer properties thereby demonstrating great potential for therapeutical applications.

The Hederagenin glycosides according to formula (I) forming component (a) of the present invention are obtainable by extraction of plant material derived from plants belonging to the genera of *Sapindus, Aesculus, Hedera, Blighia, Dipsacus, Aralia* and/or *Anemone* or mixtures thereof.

### Extracts

Another object of the present invention refers to an extract comprising one or more Hederagenin glycosides obtainable by aqueous and/or alcoholic extraction, said compounds and the respected plant or microorganisms from which they are obtained from, selected from the group consisting of:
*I.Sapindus,*
*II.Aesculus,*
*III.Hedera,*
*IV.Blighia,*
*V.Dipsacus,*
*VI.Aralia* and/or
*VII.Anemone*

The extracts according to the present invention may be prepared by methods known per se, i.e. for example by aqueous, alcoholic or aqueous/alcoholic extraction of the plants or parts thereof. Suitable extraction processes are any conventional extraction processes, such as maceration, re-maceration, digestion, agitation maceration, vortex extraction, ultrasonic extraction, counter current extraction, percolation, re-percolation, evacolation (extraction under reduced pressure), diacolation and solid/liquid extraction under continuous reflux. Percolation is advantageous for industrial use.

The plant materials that are useful for obtaining the extracts may include parts of the whole plant selected from the group consisting of blossoms, fruits, buds, roots, stems, bark, seeds and/or leaves or the whole plant itself. The starting material may be mechanically size-reduced before the extraction process. Any size reduction methods known to the expert, for example freeze grinding, may be used. Preferred solvents for the extraction process are organic solvents, water (preferably hot water with a temperature above 80 °C and more particularly above 95 °C or mixtures of organic solvents and water, more particularly low molecular weight alcohols with more or less high water contents. Extraction with methanol, ethanol and water-containing mixtures thereof is particularly preferred. The extraction process is generally carried out at about 20 to about 100 °C and preferably at about 30 to about 70 °C. In one preferred embodiment, the extraction process is carried out in an inert gas atmosphere to avoid oxidation of the ingredients of the extract. This is particularly important where extraction is carried out at temperatures above 40 °C. The extraction times are selected by the expert in dependence upon the starting material, the extraction process, the extraction temperature and the ratio of solvent to raw material, etc. After the extraction process, the crude extracts obtained may optionally be subjected to other typical steps, such as for example purification, concentration and/or decoloration. If desired, the extracts thus prepared may be subjected, for example, to the selective removal of individual unwanted ingredients. The extraction process may be carried out to any degree, but is usually continued to exhaustion. Typical yields (=extract dry matter, based on the quantity of raw material used) in the extraction of the starting materials are of the order of about 5 to about 35, %, preferably about 10 to about 30 and more preferably about 15 to about 25 % b.w. - calculated on the starting materials.

Another objective of the present invention is directed to an extract obtainable from plant material of the plant genera mentioned above, which produce a whole group of hederagenin glycosides according to formula (I).

A preferred method for preparing the Hederagenin glycosides according to formula (I) includes the following steps:
(a) providing a source of monodesmosidic hederagenin-3-glycosides;
(b) providing a solvent selected from the group consisting of water, C1 to C4 alcohols, acetone, methyl-ethyl-ketone or mixtures thereof;
(c) bringing the Hederagenin glycosides according to step (a) in contact with said solvent or solvent mixture according to step (b), optionally
   (c1) adjusting the pH value of the mixture thus obtained to about 10 to about 14 and/or
   (c2) heating the mixture thus obtained to a temperature of from about 40°C to about 90°C and/or
   (c3) extracting the hederagenin glycosides from the mixture thus obtained with n-butanol or by adsorption on a resin followed by elution with methanol to get an extract enriched with hederagenin glycosides

to obtain an extract,
wherein said source of monodesmosidic hederagenin-3-glycosides is a plant material derived from plants belonging to the genera of
   I. Sapindus,
   II. Aesculus,
   III. Hedera,
   IV. Blighia,
   V. Dipsacus,
   VI. Aralia and/or
   VII. Anemone
or mixtures thereof

Heating the extract to about 40°C to 90°C and adjusting the pH value to about 10 to about 14 allow hydrolyzing an additional sugar chain attached to the COOH group at the C-28 of hederagenin. This method also hydrolyses small acids like acetic acid bound as an ester at some of the sugar moieties. The alkaline hydrolysis of the extract enhances its antimicrobial activity.

Triterpene glycosides according to the present invention are known to possess excellent emulsifying and surfactant properties. Therefore, in a preferred embodiment, the triterpene glycosides according to the present invention substitute or support emulsifiers and surfactant present in compositions according to the present invention.

### Preferred Hederagenin glycosides

In a preferred embodiment the Hederagenin glycosides according to the present invention are selected from the following table compiling structures A to C.

| **Compound** | **Name** | **Structure** |
|---|---|---|
| A | Sapindoside B [30994-75-3] | 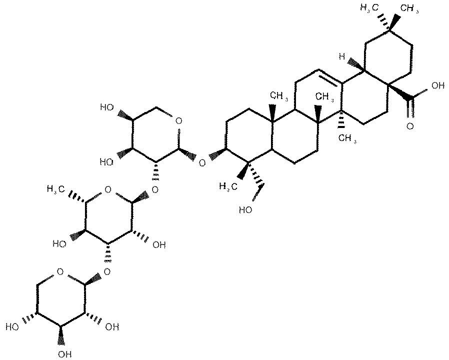 |
| B | Clemontano-side C [80666-65-5] | 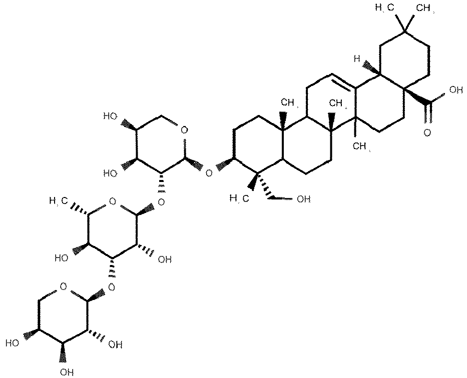 |
| C | Transsylva-noside H [174423-50-8] | 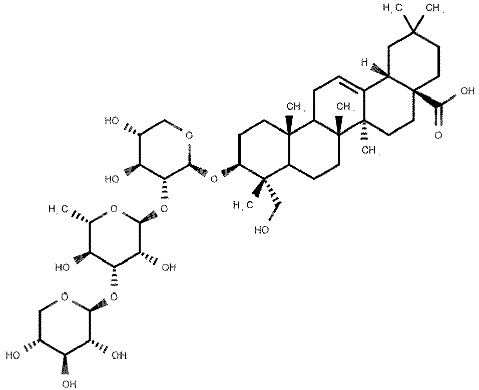 |

The composition according to the present invention may include mixtures of compounds following formulae A to C of said hederagenin glycosides or mixtures thereof or a salt of said hederagenin glycosides.

In a preferred embodiment the salts according to the present invention represent alkali salts, alkaline salts, alkaline earth salts, ammonium salts, alkanol ammonium salts, glucammo-nium salts or mixtures thereof.

In a preferred embodiment at least one hederagenin glycoside according to the present invention is added in a working amount of from 1 to 1,000 ppm - calculated on composition.

In a preferred embodiment at least one hederagenin glycoside according to the present invention is added in a working amount of from 10 to 200 ppm - calculated on composition.

In a preferred embodiment Hederagenin glycosides are added in amounts ranging from 0.0000001 to 1.0 wt.-percent - calculated on the total composition, more preferably in amounts ranging from 0.000001 to 0.1 wt.-percent, and most preferably in amounts ranging from 0.00001 to 0.01 wt.-percent.

In a preferred embodiment of the method the pH value of the composition is adjusted by inorganic or organic salts.

### Secondary additional anti-microbial compounds

In a preferred embodiment the preservative method according to the present invention may include blends of said hederagenin glycosides and additional antimicrobial compounds (component b) different from said hederagenin glycosides as they are for example listed in REGULATION (EC) No 1333/2008 OF THE EUROPEAN PARLIAMENT AND OF THE COUNCIL of 16 December 2008 on food additives or in REGULATION (EC) No 1223/2009 OF THE EUROPEAN PARLIAMENT AND OF THE COUNCIL of 30 November 2009 on cosmetic products as well as further compounds affecting solubility of said hederagenin glycosides such as saponins or emulsifiers listed in EU 1333/2008 or EU 1223/2009.

Suitable species can be selected from the group consisting of preservatives selected from the group consisting of benzoic acid and para-hydroxybenzoic acid, their esters and salts, benzyl benzoate, propionic acid and its salts, salicylic acid and its salts, 2,4-hexadienoic acid (sorbic acid) and its salts, levulinic acid and its salts, anisic acid and its salts, perillic acid and its salts, cinnamic acid and its salts, formaldehyde and paraformaldehyde, 4-hydroxy benzaldehyde, ortho-, meta-, and para-anisic aldehyde, cinnamic aldehyde, cinnamic alcohol, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexa-hydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexamethylenediguanide) hydrochloride, (Benzyloxymethoxy)-methanol hexamethylenetetramine, 1-(3-chloroallyl)-3,5,7-triaza-1-azo-nia-adamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, 1,2-dibromo-2,4-dicya-nobutane, 2,2'-methylene-bis(6-bromo-4-chlorophenol), bromochlorophene, mixture of 5-chloro-2-methyl-3(2H)-isothiazolinone, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 3-(4-Chlorphenoxy)-1,2-propanediol (Chlorphenesin), 2-chloroacetamide, chlorhexidine, chlorhexidine acetate, chlorhexidine gluconate, chlorhexidine hydrochloride, N-alkyl(C₁₂-C₂₂)trimethyl-ammonium bromide and chloride, 4,4-dimethyl-1,3-oxazol-idine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-(C₈-C₁₈)-dimethyl-benzyl-ammonium chloride, alkyl-(C₈-C₁₈)-dimethyl-benzylammonium bromide, alkyl-(C₈-C₁₈)-dimethyl-benzyl-ammonium saccharinate, benzyl hemiformal, 3-iodo-2-propynyl butylcarbamate, sodium hydroxymethyl-aminoacetate or sodium hydroxymethyl-aminoacetate, imidazolidinylurea, diazolidinylurea, sodium hydroxymethylglycinate, DMDM hydantoin, Tropolone, (Ethylendioxy)dimethanol, 2-Brom-2-(brommethyl)pentandinitril, N-(3-Aminopropyl)-N-dodecylpropan-1,3-diamin, α,α',α"-trimethyl-1,3,5-triazine-1,3,5(2H,4H,6H)-triethanol, pyridine-2-thiol-1-oxide, sodium salt, Tetrahydro-1,3,4,6-tetrakis(hydroxymethyl)imidazo[4,5-d]10erillart-2,5(1H,3H)-dion, 1,3-bis(hydroxymethyl) -1-(1,3,4-tris(hydroxy-methyl)-2,5-diox-oimidazolidin-4-yl)urea (Diazolidinyl Urea), 1,3-Bis(hydroxy-methyl)-5,5-dimethylimidazoli-dine-2,4-dione, 3-Acetyl-2-hydroxy-6-methyl-4H-pyran-4-one, cetyl pyridium chloride, caprylhydroxamic acid, sorbohydroxamic acid and their mixtures; 1,3-propanediol, methyl propanediol, 1,2-pentanediol, 1,2-hexanediol, 1,2-octanediol, 1,2-decanediol, 1,5-pentanediol, 1,6-hexanediol, 1,8-octanediol, 1,2-decanediol, ethylhexylglycerin, hexoxy-propan-1,2-diol, heptoxy- propan-1,2-diol, octoxy-propan-1,2-diol, 3-phenoxy-propan-1,2-diol, 3-benzyloxy-propan-1,2-diol, 3-phenylethyloxy-propan-1,2-diol, 3-phenylpropyloxy-propan-1,2-diol, 3-methylbenzyloxy-propan-1,2-diol, sorbitan caprylate, triclosan, climbazole, Octopirox (1-hydroxy-4-methyl-6-(2,4,4-trimethylpentyl)-2(1H)-pyridone, 2-aminoethanol), chitosan, farnesol, 2-butyloctanoic acid, 2-Benzylheptan-1-ol, glycerol monolaurate, bis(2-pyridylthio)zinc 1,1'-dioxide, N,N'-(decane-1,10-diyldipyridin-1-yl-4-ylidene)-dioctan-1-amine dihydrochloride (octenidine dihydrochloride), thymol, eugenol, benzyl alcohol, 2-phenyethyl alcohol, 3-phenyl propanol, 2-phenoxyethanol, 1-phenoxy-propan-2-ol, 3-phenoxypropanol, benzyloxymethanol, 4-hydroxyacetophenone, lactic acid, ethanol, PHB (esters). and mixtures thereof.

In a preferred embodiment, secondary additional anti-microbial compounds are selected from the group of benzoic acid and 2,4-hexadienoic acid (sorbic acid), lactic acid, ethanol and para-hydroxybenzoic acid (PHB), their esters and salts.

The preservative compositions may include components a:b in ratios by weight ranging from about 0.1:99.9 to about 99:1, preferably from about 1:99 to about 10:90, more preferably from about 20:80 to about 80:20 and most preferably from about 50:50 to about 60:40.

### FOOD PREPARATIONS

Food compositions according to the invention are any preparations or compositions comprising the compositions according to the invention which are suitable for consumption and are used for nutrition or enjoyment purposes, and are generally products which are intended to be introduced into the human or animal oral cavity, to remain there for a certain time and then either be eaten (e.g. ready-to-eat foodstuffs or feeds, see also herein below) or removed from the oral cavity again (e.g. chewing gums). Such products include any substances or products which in the processed, partially processed or unprocessed state are to be ingested by humans or animals. They also include substances which are added to orally consumable products during their manufacture, preparation or treatment and which are intended to be introduced into the human or animal oral cavity.

The food compositions according to the invention also include substances which in the unchanged, treated or prepared state are to be swallowed by a human or animal and then digested; in this respect, the orally consumable products according to the invention also include casings, coatings or other encapsulations which are to be swallowed at the same time or which may be expected to be swallowed. The expression "orally consumable product" covers ready-to-eat foodstuffs and feeds, that is to say foodstuffs or feeds that are already complete in terms of the substances that are important for the taste. The expressions "ready-to-eat foodstuff" and "ready-to-eat feed" also include solid or semi-solid ready-to-eat foodstuffs or feeds. Examples which may be mentioned are frozen products, which must be thawed and heated to eating temperature before they are eaten. Products such as yoghurt or ice-cream as well as chewing gums or hard caramels are also included among the ready-to-eat foodstuffs or feeds.

Preferred food compositions according to the invention also include "semi-finished products". Within the context of the present text, a semi-finished product is to be understood as being an orally consumable product which, because of a very high content of flavorings and taste-imparting substances, is unsuitable for use as a ready-to-eat orally consumable product (in particular foodstuff or feed). Only by mixing with at least one further constituent (e.g. by reducing the concentration of the flavorings and taste-imparting substances in question) and optionally further process steps (e.g. heating, freezing) is the semi-finished product converted into a ready-to-eat orally consumable product (in particular foodstuff or feed). Examples of semi-finished products which may be mentioned here are

Food composition according to the invention preferably comprises one or more preparations for nutrition or enjoyment purposes. These include in particular (reduced-calorie) baked goods (e.g. bread, dry biscuits, cakes, other baked articles), confectionery (e.g. chocolates, chocolate bars, other products in bar form, fruit gums, dragées, hard and soft caramels, chewing gum), meat products (e.g. ham, fresh sausage or raw sausage preparations, spiced or marinated fresh or salt meat products), eggs or egg products (dried egg, egg white, egg yolk), cereal products (e.g. breakfast cereals, muesli bars, precooked ready-to-eat rice products), dairy products (e.g. rice pudding, yoghurt, kefir, cream cheese, soft cheese, hard cheese, dried milk powder, whey, butter, buttermilk, partially or completely hydrolysed milk-protein-containing products), products made from soy protein or other soybean fractions (e.g. soy milk and products produced therefrom, preparations containing soy lecithin, fermented products such as tofu or tempeh or products produced therefrom and mixtures with fruit preparations and optionally flavors), fruit preparations (e.g. jams, sorbets, fruit sauces, fruit fillings), vegetable preparations (e.g. ketchup, sauces, dried vegetables, frozen vegetables, precooked vegetables, boiled-down vegetables), snacks (e.g. baked or fried potato crisps or potato dough products, maize- or groundnut-based extrudates), fat- and oil-based products or emulsions thereof (e.g. mayonnaise, remoulade, dressings, in each case full-fat or reduced-fat), other ready-made dishes and soups (e.g. dried soups, instant soups, precooked soups), spices, spice mixtures and in particular seasonings which are used, for example, in the snacks field, sweetener preparations, tablets or sachets, other preparations for sweetening or whitening foods. The preparations within the scope of the invention can also be used in the form of semi-finished products for the production of further preparations for nutrition or enjoyment purposes. The preparations within the scope of the invention can also be in the form of capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes, or in the form of other preparations which can be swallowed or chewed, and in the form of food supplements.

The preparations can also be in the form of capsules, tablets (uncoated and coated tablets, e.g., enteric coatings), dragées, granules, pellets, solids mixtures, dispersions in liquid phases, in the form of emulsions, in the form of powders, in the form of solutions, in the form of pastes, or in the form of other preparations which can be swallowed or chewed, for example in the form of food supplements.

The semi-finished products are generally used for the production of ready-to-use or ready-to-eat preparations for nutrition or enjoyment purposes.

Further constituents of a ready-to-eat preparation or semi-finished product for nutrition or enjoyment purposes can be conventional base substances, auxiliary substances and additives for foods or enjoyment foods, for example water, mixtures of fresh or processed, vegetable or animal base or raw substances (e.g. raw, roast, dried, fermented, smoked and/or boiled meat, bone, cartilage, fish, vegetables, herbs, nuts, vegetable pastes or mixtures thereof), digestible or non-digestible carbohydrates (e.g. sucrose, maltose, fructose, glucose, dextrins, amylose, amylopectin, inulin, xylans, cellulose, tagatose), sugar alcohols (e.g. sorbitol, erythritol), natural or hardened fats (e.g. tallow, lard, palm fat, cocoa fat, hardened vegetable fat), oils (e.g. sunflower oil, groundnut oil, maize germ oil, olive oil, fish oil, soya oil, sesame oil), fatty acids or their salts (e.g. potassium stearate), proteinogenic or non-proteinogenic amino acids and related compounds (e.g. γ-aminobutyric acid, taurine), peptides (e.g. glutathione), natural or processed proteins (e.g. gelatin), enzymes (e.g. peptidases), nucleic acids, nucleotides, taste correctors for unpleasant taste impressions, further taste modulators for further, generally not unpleasant taste impressions, other taste-modulating substances (e.g. inositol phosphate, nucleotides such as guanosine monophosphate, adenosine monophosphate or other substances such as sodium glutamate or 2-phenoxypropionic acid), emulsifiers (e.g. lecithins, diacylglycerols, gum arabic), stabilisers (e.g. carrageenan, alginate), preservatives (e.g. benzoic acid and its salts, sorbic acid and its salts), antioxidants (e.g. tocopherol, ascorbic acid), chelators (e.g. citric acid), organic or inorganic acidifying agents (e.g. acetic acid, phosphoric acid), additional bitter substances (e.g. quinine, caffeine, limonene, amarogentine, humulone, lupulone, catechols, tannins), substances that prevent enzymatic browning (e.g. sulfite, ascorbic acid), ethereal oils, plant extracts, natural or synthetic colourings or colouring pigments (e.g. carotinoids, flavonoids, anthocyans, chlorophyll and derivatives thereof), spices, trigeminally active substances or plant extracts containing such trigeminally active substances, synthetic, natural or nature-identical flavourings or odorants as well as odour correctors.

Food compositions according to the invention, for example those in the form of preparations or semi-finished products, preferably comprise a flavour composition in order to complete and refine the taste and/or odour. A preparation can comprise as constituents a solid carrier and a flavour composition. Suitable flavour compositions comprise, for example, synthetic, natural or nature-identical flavourings, odorants and taste-imparting substances, reaction flavourings, smoke flavourings or other flavour-giving preparations (e.g. protein (partial) hydrolysates, preferably protein (partial) hydrolysates having a high arginine content, barbecue flavourings, plant extracts, spices, spice preparations, vegetables and/or vegetable preparations) as well as suitable auxiliary substances and carriers. Particularly suitable here are the flavour compositions or constituents thereof which produce a roasted, meaty (in particular chicken, fish, seafood, beef, pork, lamb, mutton, goat), vegetable-like (in particular tomato, onion, garlic, celery, leek, mushroom, aubergine, seaweed), spicy (in particular black and white pepper, cardamom, nutmeg, pimento, mustard and mustard products), fried, yeast-like, boiled, fatty, salty and/or pungent flavour impression and accordingly can enhance the spicy impression. The flavour compositions generally comprise more than one of the mentioned ingredients.

The food compositions of the present invention are preferably selected from the group comprising
- confectionery, preferably reduced-calorie or calorie-free confectionery, preferably selected from the group comprising muesli bar products, fruit gums, dragées, hard caramels and chewing gum,
- cereal products, preferably selected from the group comprising low-sugar and sugar-free breakfast cereals and muesli bars,
- dairy products, preferably selected from the group comprising reduced-fat and fat-free yoghurt, kefir, whey, buttermilk and ice-cream,
- products made from soy protein or other soybean fractions, preferably selected from the group comprising soy milk, products produced from soy milk, preparations containing soy lecithin, products produced from preparations containing soy lecithin and mixtures with fruit preparations and optionally flavours,
- sweetener preparations, tablets and sachets,
- sugar-free dragées,
- ice-cream, with or without milk-based constituents, preferably sugar-free.

### Food grade emulsifiers

The preferred food grade emulsifiers encompass the following groups:
- Lecithin;
- Fatty acid derivatives, such as for example polyglycerol esters (PGE), polysorbates ("TWEEN"), stearoyl lactylates, propylene glycol esters (PGMS), and sucrose esters;
- Polyglycerol Polyricinoleate (PGPR);
- Ammonium Phosphatide (AMP);
- Mono and Diglycerides of C₆-C₂₂ saturated or unsaturated fatty acids;
- Saponins or extracts prepared from saponin-containing plants, e.g. Quillaia extract (E999)
and mixtures thereof.

### Aroma or flavoring compounds

Aroma compounds and flavoring agents are well known in the art and can be chosen from synthetic flavoring liquid and/or oils derived from plants leaves, flowers, fruits and so forth, and combinations thereof. Representative flavoring liquids include: artificial, natural or synthetic fruit flavors such as eucalyptus, lemon, orange, banana, grape, lime, apricot and grapefruit oils and fruit essences including apple, strawberry, cherry, orange, pineapple and so forth; bean and nut derived flavors such as coffee, cocoa, cola, peanut, almond and so forth; and root derived flavors such as licorice or ginger.

Suitable aroma or flavoring compounds are listed in REGULATION (EC) No 1334/2008 OF THE EUROPEAN PARLIAMENT AND OF THE COUNCIL of 16 December 2008 on flavorings and certain food ingredients with flavoring properties for use in and on foods and amending Council Regulation (EEC) No 1601/91, Regulations (EC) No 2232/96 and (EC) No 110/2008 and Directive 2000/13/EC.

### Sweeteners

The term "sweeteners" here denotes substances having a relative sweetening power of at least 25, based on the sweetening power of sucrose (which accordingly has a sweetening power of 1). Sweeteners to be used in an orally consumable product (in particular foodstuff, feed or medicament) according to the invention (a) are preferably non-cariogenic and/or have an energy content of not more than 5 kcal per gram of the orally consumable product.

Advantageous sweeteners in a preferred orally consumable product (in particular foodstuff, feed or medicament) according to the invention are selected from the following groups:
(a) Naturally occurring sweeteners, preferably selected from the group comprising miraculin, monellin, mabinlin, thaumatin, curculin, brazzein, pentaidin, D-phenylalanine, D-tryptophan, and extracts or fractions obtained from natural sources, comprising those amino acids and/or proteins, and the physiologically acceptable salts of those amino acids and/or proteins, in particular the sodium, potassium, calcium or ammonium salts, neohesperidin dihydrochalcone, naringin dihydrochalcone, stevioside, steviolbioside, rebaudiosides, in particular rebaudioside A, rebaudioside B, rebaudioside C, rebaudioside D, rebaudioside E, rebaudioside F, rebaudioside G, rebaudioside H, dulcosides and rubusoside, suavioside A, suavioside B, suavioside G, suavioside H, suavioside I, suavioside J, baiyunoside 1, baiyunoside 2, phlomisoside 1, phlomisoside 2, phlomisoside 3 and phlomisoside 4, abrusoside A, abrusoside B, abrusoside C, abrusoside D, cyclocaryoside A and cyclocaryoside I, osladin, polypodoside A, strogin 1, strogin 2, strogin 4, selligueain A, dihydroquercetin 3-acetate, perillartin, telosmoside A₁₅, periandrin I-V, pterocaryo-sides, cyclocaryosides, mukuroziocides, trans-anethole, trans-cinnamaldehyde, bryo-sides, bryonosides, bryonodulcosides, carnosiflosides, scandenosides, gypenosides, trilo-batin, phloridzin, dihydroflavanols, hematoxylin, cyanin, chlorogenic acid, albiziasaponin, telosmosides, gaudichaudioside, mogrosides, mogroside V, hernandulcins, monatin, phyllodulcin, glycyrrhetinic acid and derivatives thereof, in particular glycosides thereof such as glycyrrhizine, and the physiologically acceptable salts of those compounds, in particular the sodium, potassium, calcium or ammonium salts; and extracts or concentrated fractions of the extracts, selected from the group comprising thaumatococcus extracts (katamfe plant), extracts from *Stevia* ssp. (in particular *Stevia rebaudiana*), swingle extracts (*Momordica* or *Siratia grosvenorii,* Luo-Han-Guo), extracts from *Glycerrhyzia* ssp. (in particular *Glycerrhyzia glabra*), extracts from *Rubus* ssp. (in particular *Rubus suavissimus*), citrus extracts and extracts from *Lippia dulcis;*
(b) Synthetic sweet-tasting substances, preferably selected from the group comprising magap, sodium cyclamate or other physiologically acceptable salts of cyclamic acid, acesulfame K or other physiologically acceptable salts of acesulfame, neohesperidin dihydrochalcone, naringin dihydrochalcone, saccharin, saccharin sodium salt, aspartame, superaspartame, neotame, alitame, advantame, perillartin, sucralose, lugduname, carrelame, sucrononate and sucrooctate.

### Thickeners

Advantageous thickeners in a preferred orally consumable product (in particular foodstuff, feed or medicament) according to the invention are selected from the group comprising: crosslinked polyacrylic acids and derivatives thereof, polysaccharides and derivatives thereof, such as xanthan gum, agar-agar, alginates or tyloses, cellulose derivatives, for example carboxymethylcellulose or hydroxycarboxymethylcellulose, fatty alcohols, monoglycerides and fatty acids, polyvinyl alcohol and polyvinylpyrrolidone.

Preference is given according to the invention to an orally consumable product (in particular foodstuff or feed) which comprises milk thickened with lactic acid bacteria and/or cream thickened with lactic acid bacteria and which preferably is selected from the group comprising yoghurt, kefir and quark.

A food composition according to the invention comprising milk thickened with lactic acid bacteria and/or cream thickened with lactic acid bacteria is advantageously an orally consumable product which comprises a probiotic, wherein the probiotic is preferably selected from the group comprising Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) and Lactobacillus rhamnosus (ATCC 53013).

### Additives for chewing gums

Particular preference is given to an orally consumable product (in particular foodstuff, feed or medicament) according to the invention that is a chewing gum and comprises a chewing-gum base. The chewing-gum base is preferably selected from the group comprising chewing-gum or bubble-gum bases. The latter are softer, so that gum bubbles can also be formed therewith. Preferred chewing-gum bases according to the invention include, in addition to the natural resins or the natural latex chicle that are traditionally used, elastomers such as polyvinyl acetate (PVA), polyethylene, (low or medium molecular weight) polyisobutene (PIB), polybutadiene, isobutene-isoprene copolymers (butyl rubber), polyvinyethyl ether (PVE), polyvinylbutyl ether, copolymers of vinyl esters and vinyl ethers, styrene-butadiene copolymers (styrene-butadiene rubber, SBR) or vinyl elastomers, for example based on vinyl acetate/vinyl laurate, vinyl acetate/vinyl stearate or ethylene/vinyl acetate, as well as mixtures of the mentioned elastomers, as described, for example, in EP 0 242 325, US 4,518,615, US 5,093,136, US 5,266,336, US 5,601,858 or US 6,986,709. In addition, chewing-gum bases that are preferably to be used according to the invention preferably comprise further constituents such as, for example, (mineral) fillers, plasticisers, emulsifiers, antioxidants, waxes, fats or fatty oils, such as, for example, hardened (hydrogenated) vegetable or animal fats, mono-, di- or tri-glycerides. Suitable (mineral) fillers are, for example, calcium carbonate, titanium dioxide, silicon dioxide, talcum, aluminium oxide, dicalcium phosphate, tricalcium phosphate, magnesium hydroxide and mixtures thereof. Suitable plasticisers, or agents for preventing adhesion (detackifiers), are, for example, lanolin, stearic acid, sodium stearate, ethyl acetate, diacetin (glycerol diacetate), triacetin (glycerol triacetate), triethyl citrate. Suitable waxes are, for example, paraffin waxes, candelilla wax, carnauba wax, microcrystalline waxes and polyethylene waxes. Suitable emulsifiers are, for example, phosphatides such as lecithin, mono- and di-glycerides of fatty acids, for example glycerol monostearate.

Chewing gums according to the invention (in particular as disclosed above) preferably comprise constituents such as sugars of different types, sugar substitutes, other sweet-tasting substances, sugar alcohols (in particular sorbitol, xylitol, mannitol), ingredients having a cooling effect, taste correctors for unpleasant taste impressions, further taste-modulating substances (e.g. inositol phosphate, nucleotides such as guanosine monophosphate, adenosine monophosphate or other substances such as sodium glutamate or 2-phenoxypropionic acid), humectants, thickeners, emulsifiers, stabilisers, odour correctors and flavours (e.g. eucalyptusmenthol, cherry, strawberry, grapefruit, vanilla, banana, citrus, peach, blackcurrant, tropical fruits, ginger, coffee, cinnamon, combinations (of the mentioned flavours) with mint flavours as well as spearmint and peppermint on their own). The combination *inter alia* of the flavours with further substances that have cooling, warming and/or mouth-watering properties is of particular interest.

### COSMETIC PREPARATIONS

Another object of the present invention refers to a skin care, personal care, sun care or hair care product or product formulation, comprising the composition as defined above. The preparations may represent for example a cosmetic cream, lotion, spray, emulsion, ointment, gel or mouse and the like.

The preparations according to the invention may contain antidandruff agents, irritation-preventing agents, irritation-inhibiting agents, antioxidants, adstringents, perspiration-inhibiting agents, antiseptic agents, ant-statics, binders, buffers, carrier materials, chelating agents, cell stimulants, cleansing agents, care agents, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, hair promotion agents, cooling agents, skin-cooling agents, warming agents, skin-warming agents, stabilizers, UV-absorbing agents, UV filters, detergents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, colour-protecting agents, pigments, odoriferous substances, polyols, surfactants, electrolytes, organic solvents or silicone derivatives and the like as additional auxiliaries and additives.

### INDUSTRIAL APPLICATION

Another object of the present invention refers to a method for obtaining Hederagenin glycosides according to Claim 1 encompassing following steps:
(a) providing a source of monodesmosidic hederagenin-3-glycosides;
(b) providing a solvent selected from the group consisting of water, C1 to C4 alcohols, acetone, methyl-ethyl-ketone or mixtures thereof;
(c) bringing the Hederagenin glycosides according to step (a) in contact with said solvent or solvent mixture according to step (b), optionally
   (c1) adjusting the pH value of the mixture thus obtained to about 10 to about 14 and/or
   (c2) heating the mixture thus obtained to a temperature of from about 40°C to about 90°C and/or
   (c3) extracting the hederagenin glycosides from the mixture thus obtained with n-butanol or by adsorption on a resin followed by elution with methanol to get an extract enriched with hederagenin glycosides

to obtain an extract,
wherein said source of monodesmosidic hederagenin-3-glycosides is a plant material derived from plants belonging to the genera of
   *I. Sapindus,*
   *II. Aesculus,*
   *III. Hedera,*
   *IV. Blighia,*
   *V. Dipsacus,*
   *VI. Aralia* and/or
   *VII. Anemone*
or mixtures thereof.

Another object of the present invention refers to a method for improving antimicrobial stability of a cosmetic, detergent or food preparation encompassing the following steps:
a) providing a preparation with low or no antimicrobial stability and
b) adding a working amount of the preservative composition according to the present invention.

A preferred embodiment is a cosmetic, detergent and food preparation comprising the preservative composition according to the present invention.

Also, object of the present invention is the use of the preservative compositions according to the present invention for antimicrobial stabilization of cosmetic, detergent or food preparations excluding beverages.

For the sake of good order, it is emphasized that all preferred embodiments mentioned above, particularly with respect to combinations, ratios and amounts also apply to compositions, preparations, methods and the use as claimed. Therefore, their repetition is not necessary.

### EXAMPLES

### MANUFACTURING EXAMPLES

### Example 1: Isolation of monodesmosidc hederagenin glycosides

### A) Extraction, isolation and identification of compounds of formula A to C

1.0 kg dried plant material was extracted subsequently with 12.51 methanol-MTB-ether (50:50 v/v) and methanol (100%) at room temperature for 24h. Both extracts were combined and the solvent evaporated in vacuum. The extract yield depends on the used plant species and the part of the plant. Typically, the extract yield is about 5 to 30% of the dried plant material.

### B) Pre-fractionation by reverse phase chromatography

The raw extract was subjected to a reverse phase medium pressure chromatography to enrich the compounds of interest. Reverse phase MPLC allows to remove sugars and lipids as well as other secondary metabolites like flavonoids very efficiently and to reach enriched fractions of compounds of interest, which could be put on a second fractions step by HPLC using finer RP material. Sometimes it makes sense, to combine different MPLC prefractionations methods to get a more specific enrichment of selected saponins, e.g. by combination of RP18 and RP4 material.

### C) Second purification step by reverse phase chromatography

Pure compounds were isolated by reverse phase chromatography using different fractions from the first separation step by MPLC. To reach a purity of >90% per compound sometimes several repeated separations by HPLC were necessary. Promising fractions from the preparative HPLC runs detected by light scattering detection ELSD were analyzed by LCMS. The solvent of all fractions containing compounds of interest was removed in vacuum followed by a freeze drying step. Isolated compounds were characterized by LCMS and 1D and 2D NMR spectroscopy.

### Example 2: Preparation of a saponin extract from Sapindus mukurossi enriched with monodesmosidic hederagenin glycosides

Dried fruit shells of *Sapindus mukurossi* were extracted methanol-water 9:1 at 40°C for 24h. The solvent was evaporated in vacuum. The extract was dissolved in 3% KOH and heated to 60°C for 1 h, neutralized by addition of HCI and chromatographed on a column of Diaion HP-20 by eluting with water and then with MeOH. The MeOH fraction was evaporated to dryness to get an extract enriched with monodesmosidic hederagenin glycosides without any esters at the monosaccharide chain.

### D) Isolated compounds

| **Compound** | **Name** | **Structure** | **Plant material** |
|---|---|---|---|
| A | Sapindosid e B [30994-75-3] | 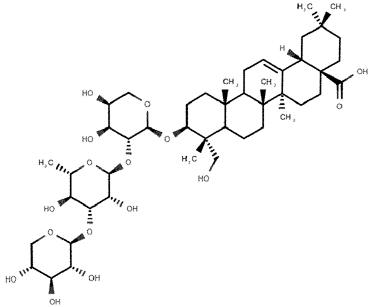 | *Sapindus mukurossi* fruits |
| B | Clemontanoside C [80666-65-5] | 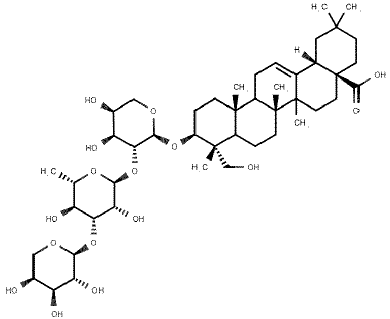 | *Anemone rivularis* roots |
| C | Transsylvanoside H [174423-50-8] | 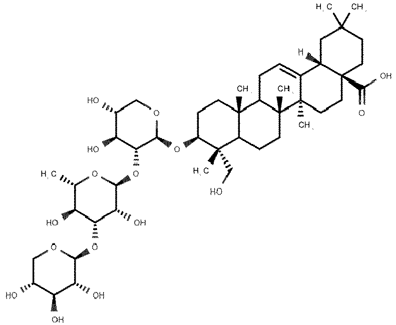 | *Clematis armandii* stem |

### Example 3: Antimicrobial activity

Compounds are dissolved at a concentration of 20mg/mL using 100% DMSO.
- Preculture: yeast and bacteria are plated on potato dextrose agar, resuspended in potato dextrose broth and CFU via OD measurement determined; adjusting CFU of the strain in PDB pH 3.8
- Preculture: for mycel forming fungi spores were directly used after thawing of cryopreserved stocks of known titre
- Final titre used for the assay: Yeast strains 5*10³ CFU/ml, Bacteria 1*10⁶ CFU/ml, Fungi (spores) 5*10⁴ CFU/ml
- Potato dextrose broth pH 3.8 was used as assay matrix
- 500ppm (0.05%) of the compounds were screened (final DMSO 2.5% (v/v))
- Volume: 100µl in 96-well format
- Positive controls: potassium sorbate, sodium benzoate & triclosan
- 28°C to 30°C, static incubation
- Incubation time depending on spoilage organism between 2 and 8 days
- Visual evaluation (per eye, for fungi with binocular magnification)

Antimicrobial activities are reported in Table 1

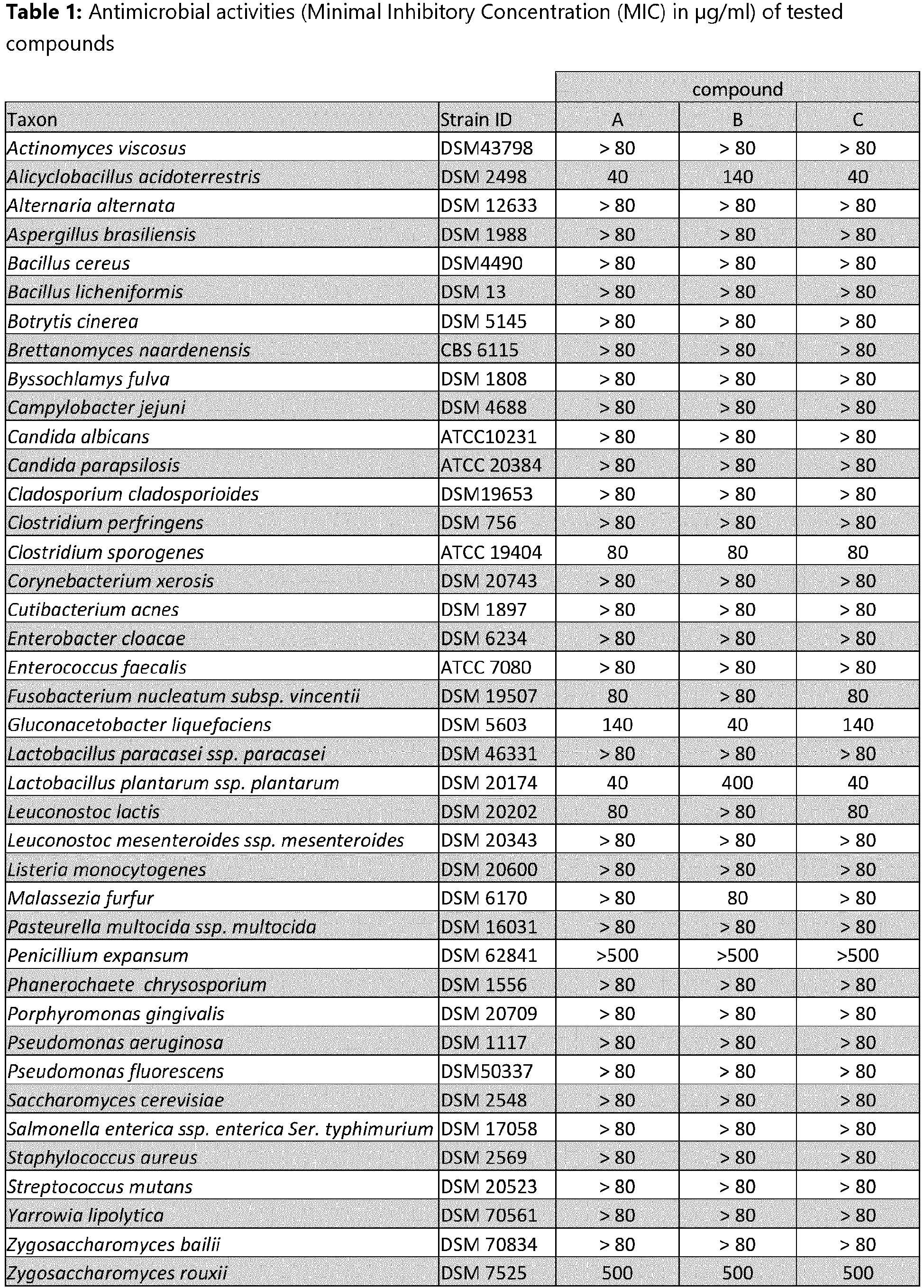

### FORMULATION EXAMPLES

### FORMULATION EXAMPLES FOOD

The following examples **F1 to F2** show various formulations for food preparations. Saponin stands for one or more hederagenin glycosides according to Claim 1.

### EXAMPLE F1

An Italian Salad dressing formulation is prepared as follows:

| Ingredient | Parts |
|---|---|
| Part I | |
| water | 70.0 |
| Vinegar | 7.0 |
| Sodium Chloride | 2.0 |
| Sweetener | 4.0 |
| Gum | 0.4 |
| Flavors | 0.9 |
| Part II | |
| Vegetable Oil | 5.3 |
| Part III | |
| Water | 6.2 |
| Vinegar | 4.2 |
| Spices and Flavors | 1.8 |

Parts 1 and 2 are mixed and homogenized. Part 3 is then added and thoroughly mixed to form a homogenous mixture. 400 ppm of saponin is added to the dressing formula.

### EXAMPLE F2: Ketchup

### aqueous phase

| | |
|---|---|
| ground tomato | 25.9 |
| cider vinegar | 20.7 |
| water | 44.3 |
| Sundried tomatoes | 2.1 |
| sodium chloride | 1.8 |
| dehydrated minced garlic | 1.1 |
| frozen oregano | 0.4 |
| basil | 0.5 |
| Xanthan gum | 0.2 |
| white onion powder | 2.5 |
| sucrose | 7.0 |
| EDTA | 0.0007 |
| saponin | 0.05 |

| **oil phase** | |
|---|---|
| soybean oil | 53.3 |
| olive oil | 46.7 |

### FORMULATION EXAMPLES PETFOOD

The following examples **P1 to P4** show various formulations for pet food preparations. Saponin stands for one or more hederagenin glycosides according to Claim 1.

### EXAMPLE P1

This example describes the preparation of a 60% moisture, microbiologically-stable food product simulating meat chunks which is suitable for use as a dog food. The food is prepared from the following ingredients:

| Ingredient | Parts |
|---|---|
| Wheat gluten | 10.0 |
| Soya bean oil meal | 4.0 |
| Poultry meal | 3.0 |
| Meat meal | 1.5 |
| Corn meal | 1.2 |
| Tallow | 5.0 |
| Carrageenan | 2.5 |
| Potassium sorbate | 0.3 |
| Tween 80 | 0.25 |
| Span 80 | 0.75 |
| Saponin | 0.5 |
| High fructose corn syrup (80% solids, 90% fructose) | 15.0 |
| Water | 56.0 |

To prepare the food, the carrageenan is added to the water at 70° C. and stirred to dissolve. The Tween 80, polyoxyethylene sorbitan monoleate available from Atlas Chemical, potassium sorbate, benzyl alcohol and the high fructose corn syrup (Isomerose 900, available from Clinton Corn Products) are added to the carrageenan solution and stirred vigorously to dissolve. The tallow is melted and the Span 80, sorbitan monooleate, available from Atlas Chemical, is added to it and mixed thoroughly. The tallow and aqueous solution are then mixed vigorously to emulsify. The remaining dry components are added with stirring to blend with the emulsion. The finished mix is added to a pan and autoclaved at 15 lbs gauge pressure for 20 minutes to heat set the mass. The heat-set cohesive product has a moisture content of 60% and is microbiologically stable. Without the glycolipid component this formulation would not be microbiologically stable.

### EXAMPLE P2

This example describes the production of a 70% moisture, microbiologically stable food product simulating meat chunks from the following ingredients:

| Ingredient | Parts |
|---|---|
| Wheat gluten | 7.48 |
| Soya bean oil meal | 2.96 |
| Poultry meal | 2.23 |
| Meat meal | 1.11 |
| Corn meal | 0.88 |
| Tallow | 3.70 |
| Carrageenan | 1.85 |
| Potassium sorbate | 0.30 |
| Span 80 | 0.75 |
| Tween 80 | 0.25 |
| Saponin | 0.50 |
| High fructose corn syrup | 10.10 |
| Water | 66.90 |

The process is the same as in Example 1.

### EXAMPLE P3

This example describes the preparation of a microbiologically-stable gravy having moisture content of 60%. The formulation is as follows:

| Ingredient | Parts |
|---|---|
| Meat & bone meal | 9.45 |
| Yeast | 1.35 |
| Corn flour | 1.35 |
| Dried whey | 1.35 |
| Whole egg solids | 1.35 |
| Tallow | 9.45 |
| High fructose corn syrup | 20.23 |
| Tween 80 | 0.25 |
| Span 80 | 0.75 |
| Saponin | 0.50 |
| Kelcosol | 0.14 |
| Water | 54.0 |

The process according to this example calls for first mixing the Span 80 with the tallow in melted condition. Next, a solution containing the high fructose corn syrup, water, potassium sorbate, Tween 80, kelcosol, sodium alginate, available from Kelco Co., and benzyl alcohol is prepared by heating the water to 60° C. and mixing. The solution is then stirred vigorously with the tallow to emulsify. The remaining dry materials are then added and stirred vigorously. No heating is necessary other than in the initial mixing of the fat and aqueous phases.

### EXAMPLE P4

This example describes the preparation of a microbiologically-stable gravy having a moisture content of 70%. The following ingredients are combined according to the process of Example 3:

| Ingredient | Parts |
|---|---|
| Meat & bone meal | 7.0 |
| Corn flour | 1.0 |
| Brewers yeast | 1.0 |
| Dried whey | 1.0 |
| Whole egg solids | 1.0 |
| Tallow | 7.0 |
| High fructose corn syrup | 15.0 |
| Kelcosol | 0.1 |
| Span 80 | 0.75 |
| Tween 80 | 0.25 |
| Saponin | 0.50 |
| Water | 65.30 |

### FORMULATION EXAMPLES COSMETICS & DETERGENTS

The following examples **F1 to F56** show various formulations for preparations. Formulation 1 stands for one or more hederagenin glycosides according to Claim 2.

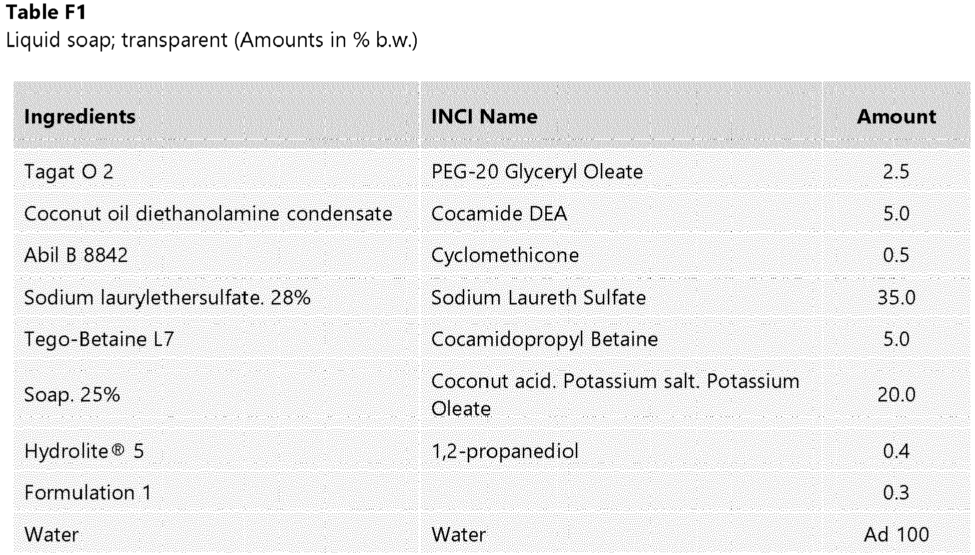

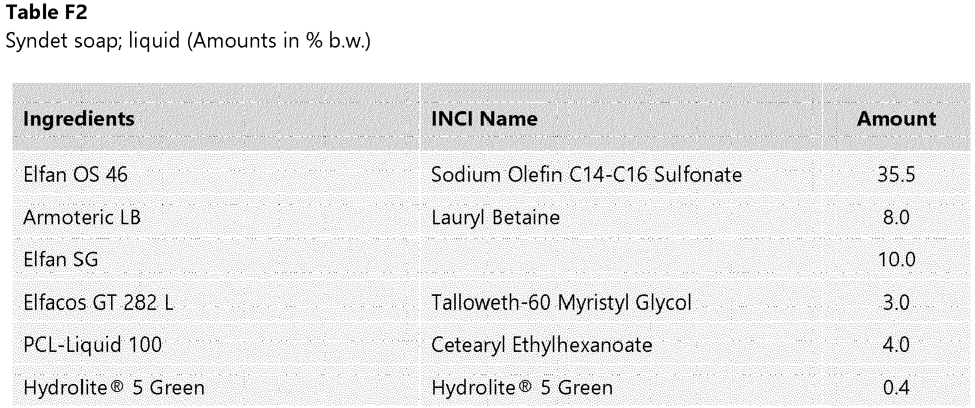

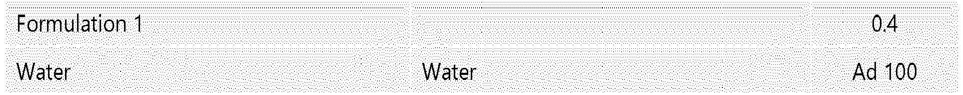

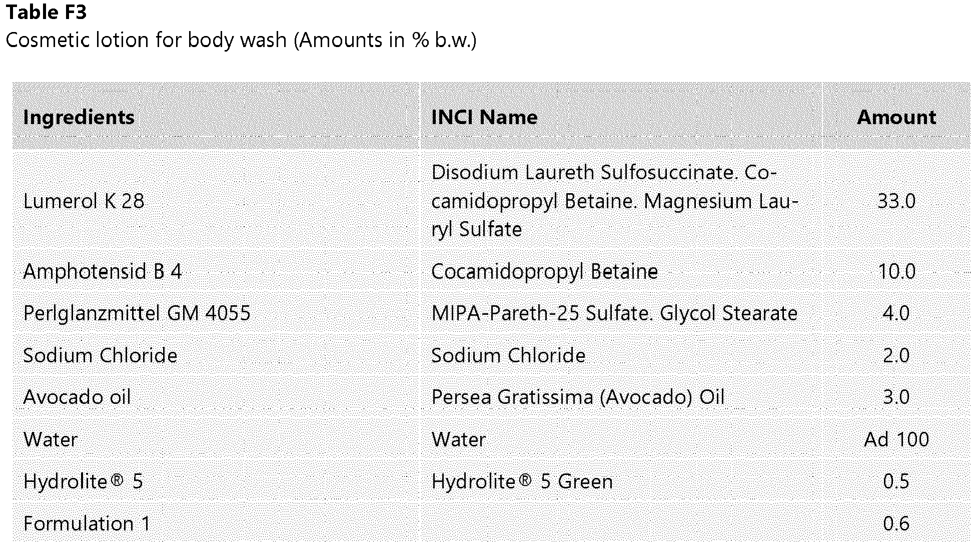

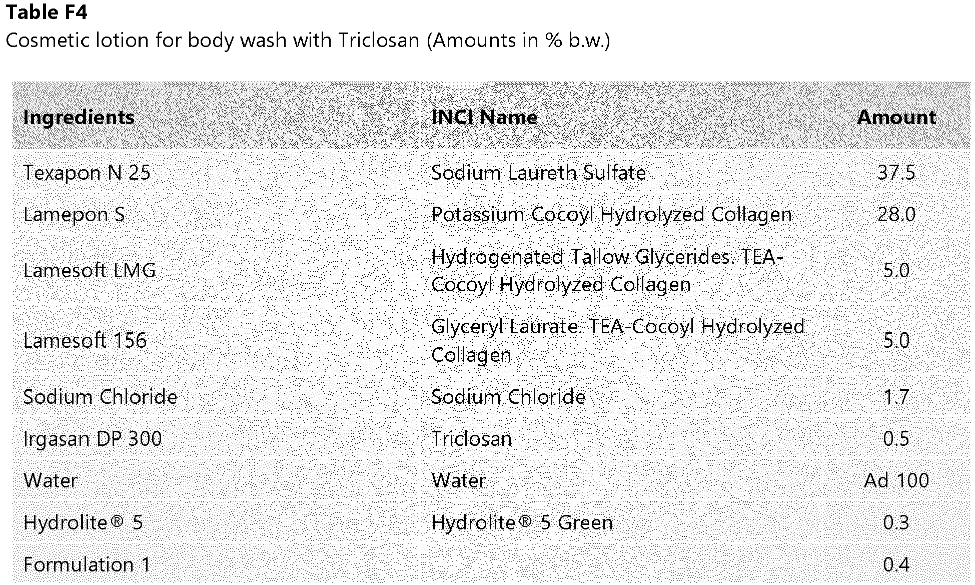

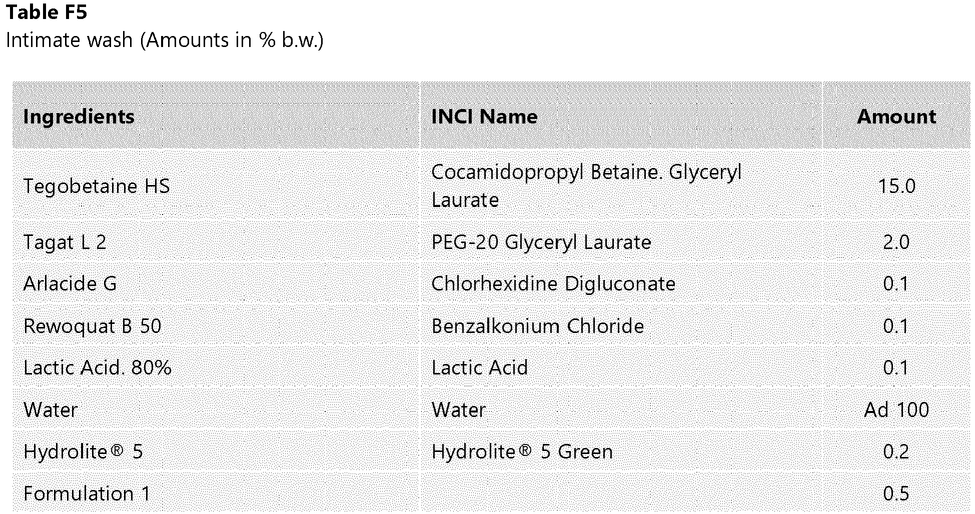

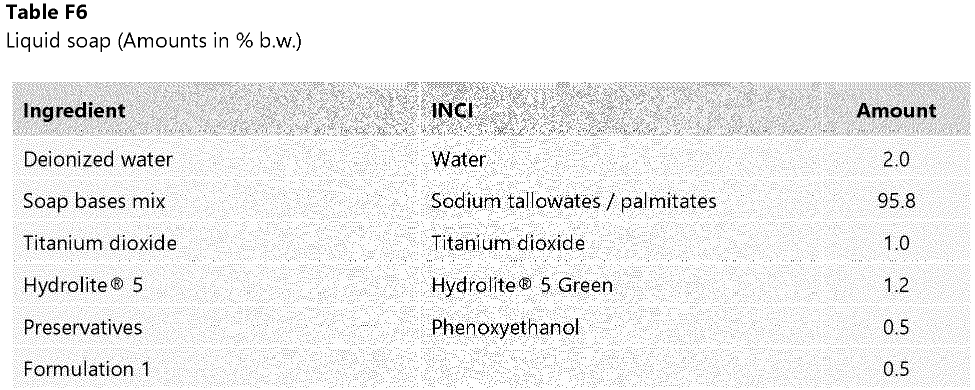

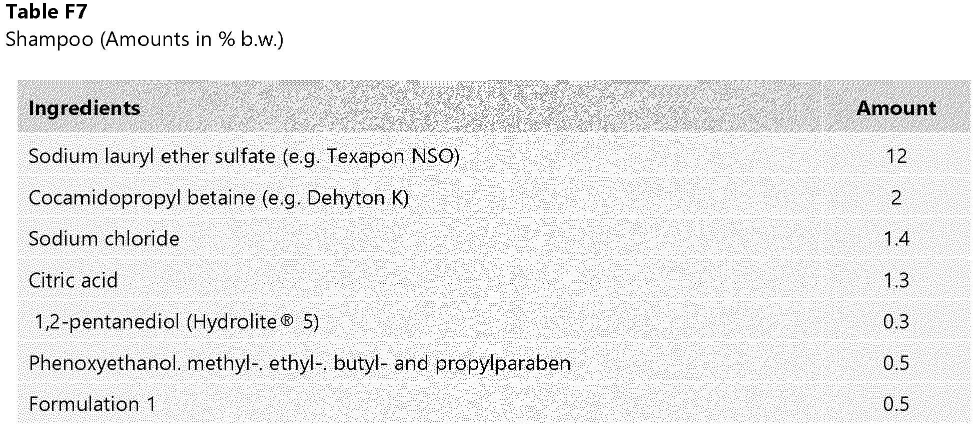

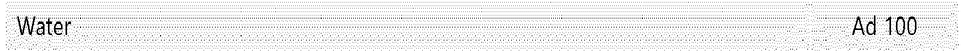

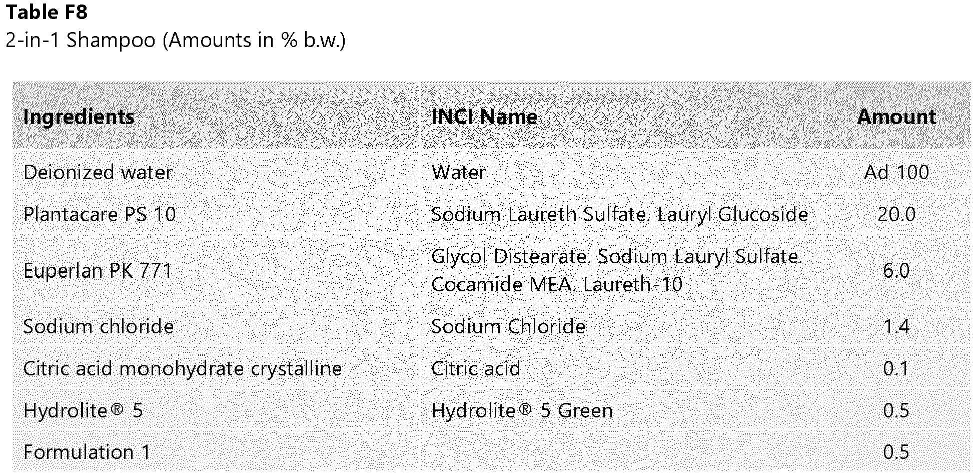

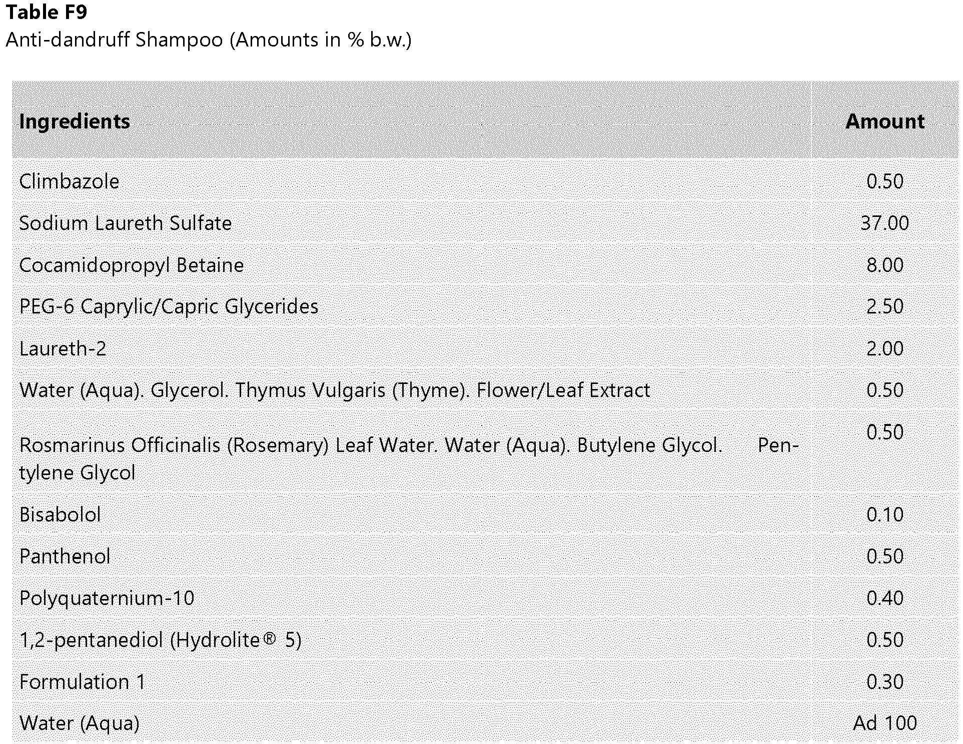

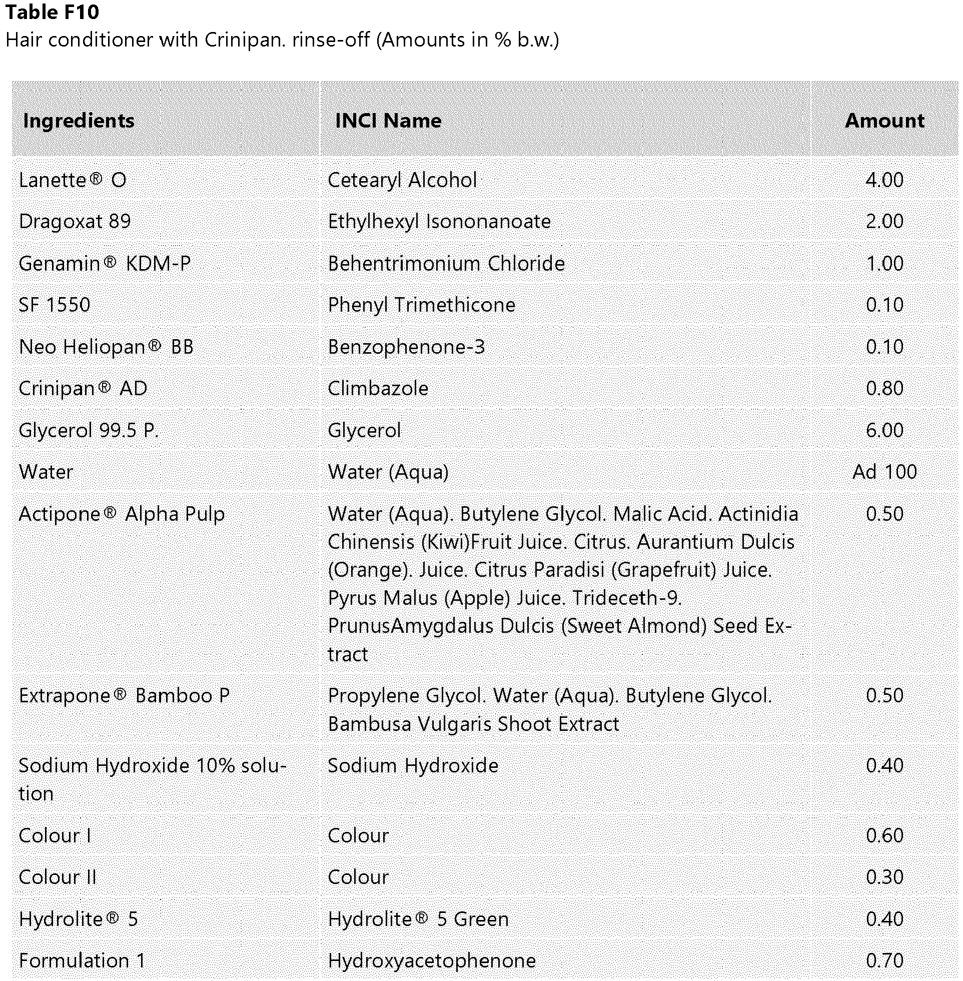

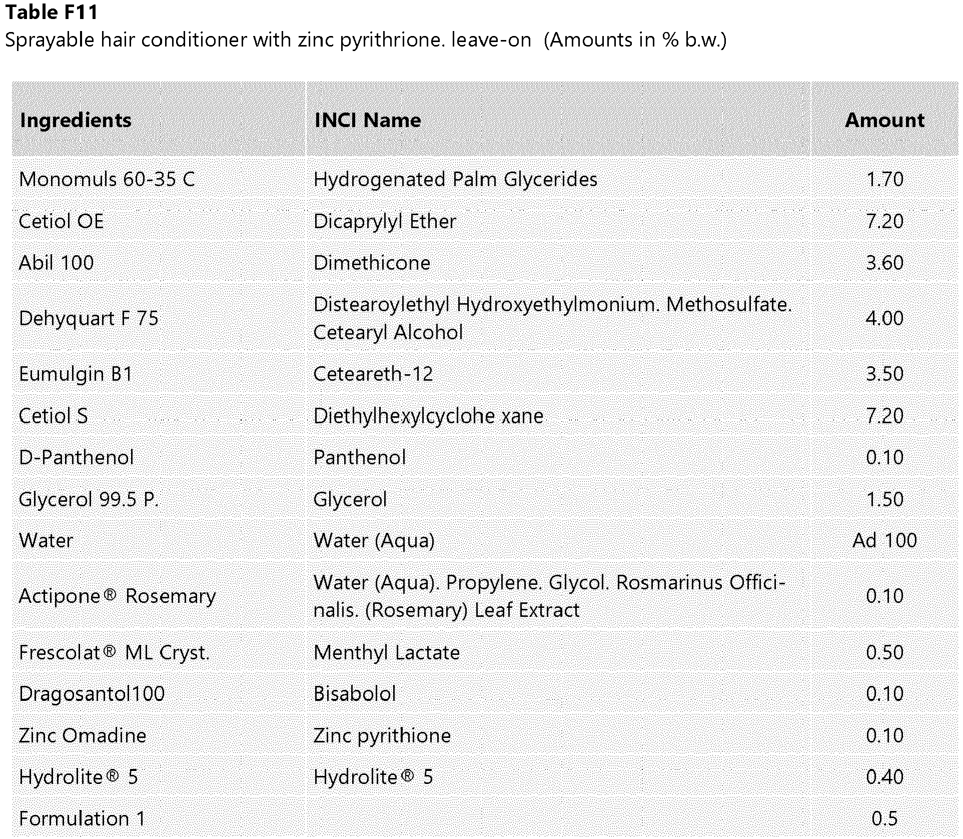

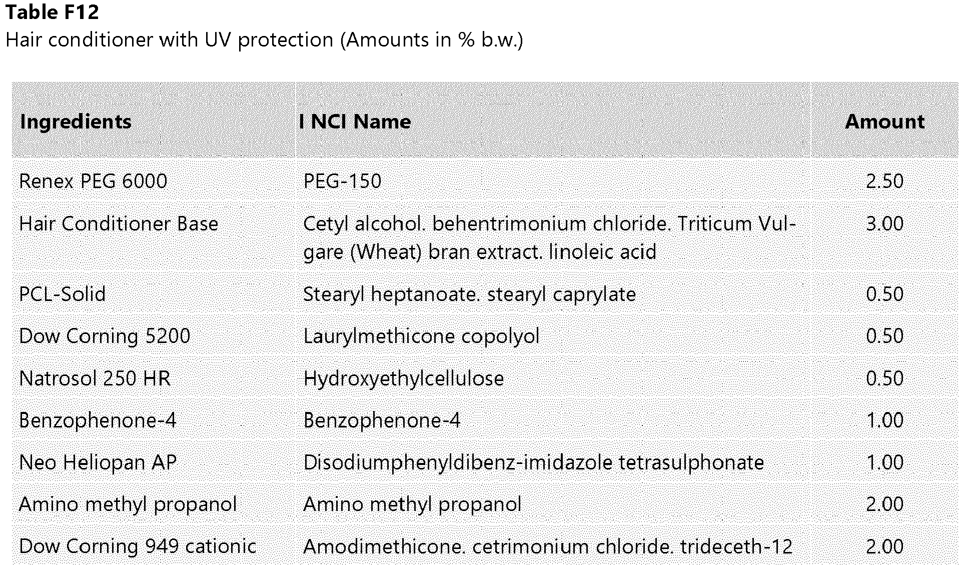

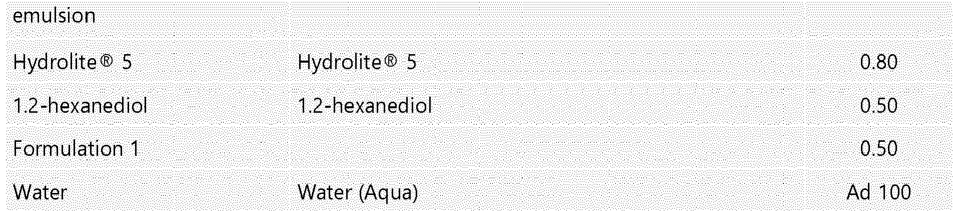

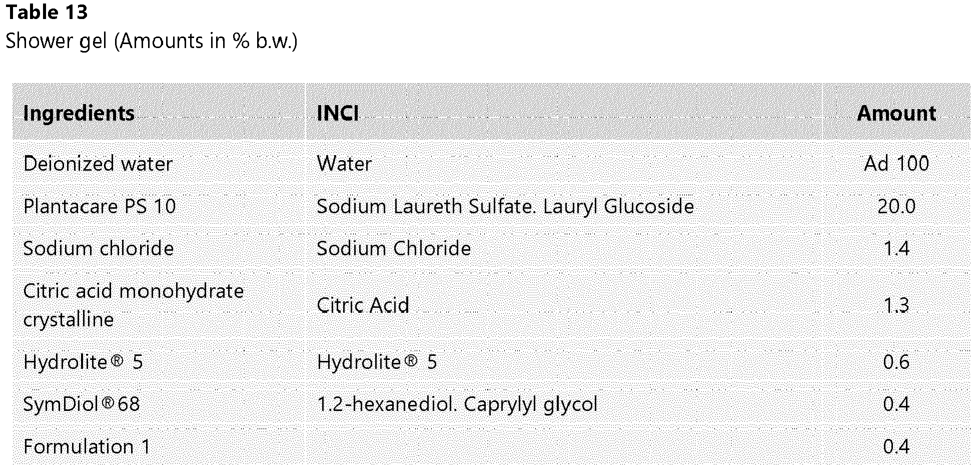

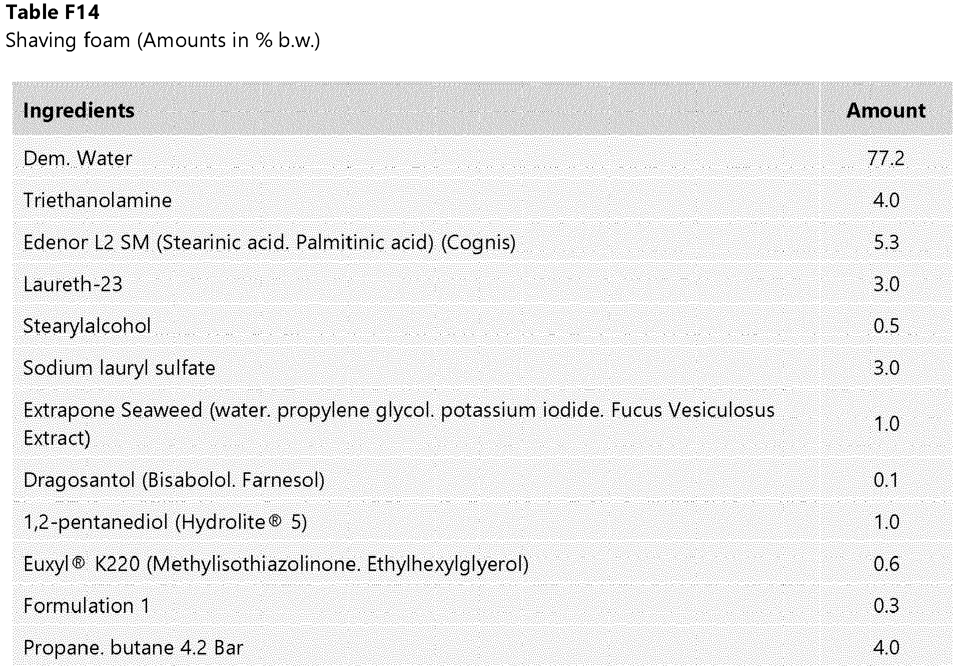

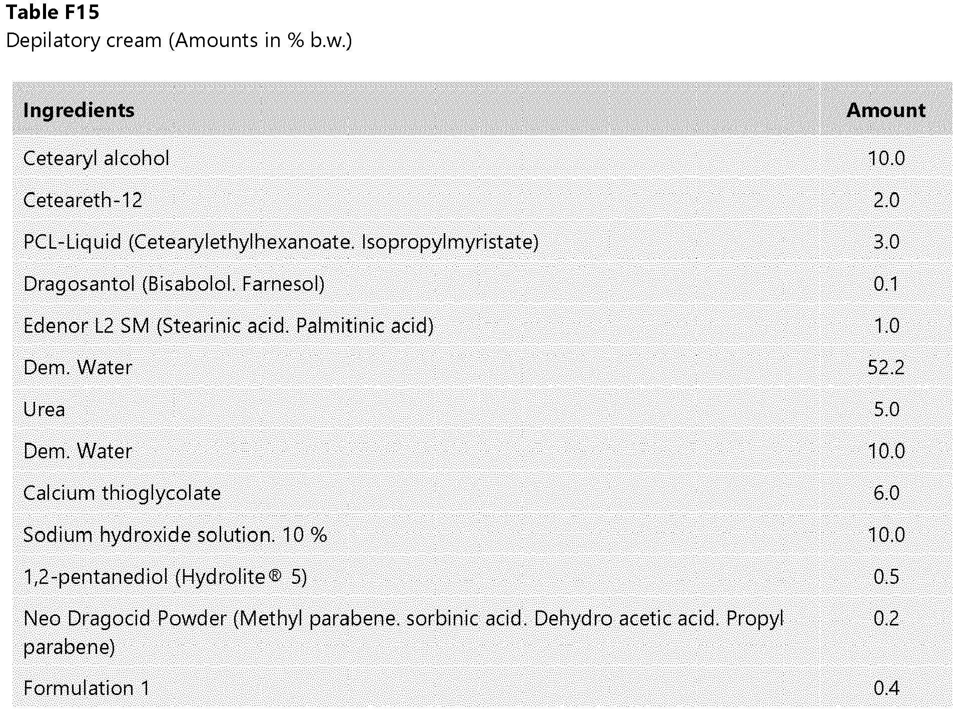

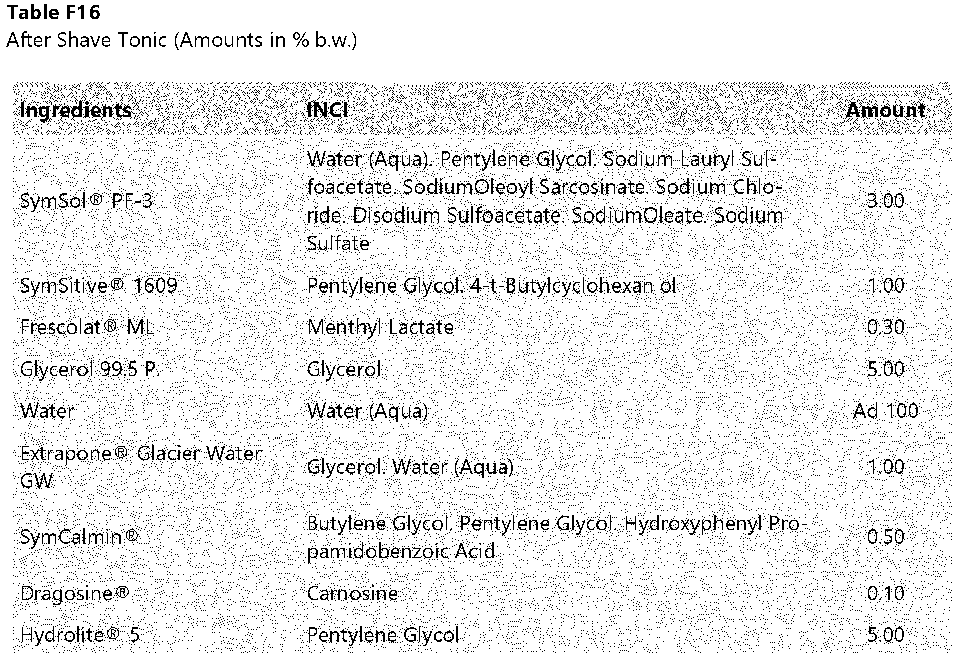

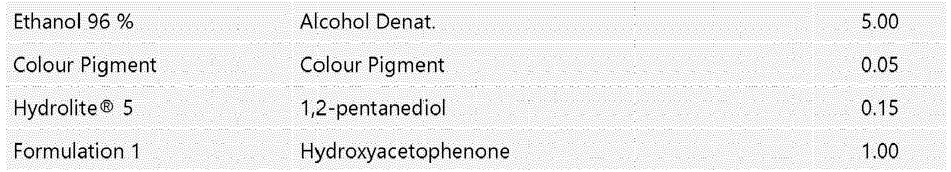

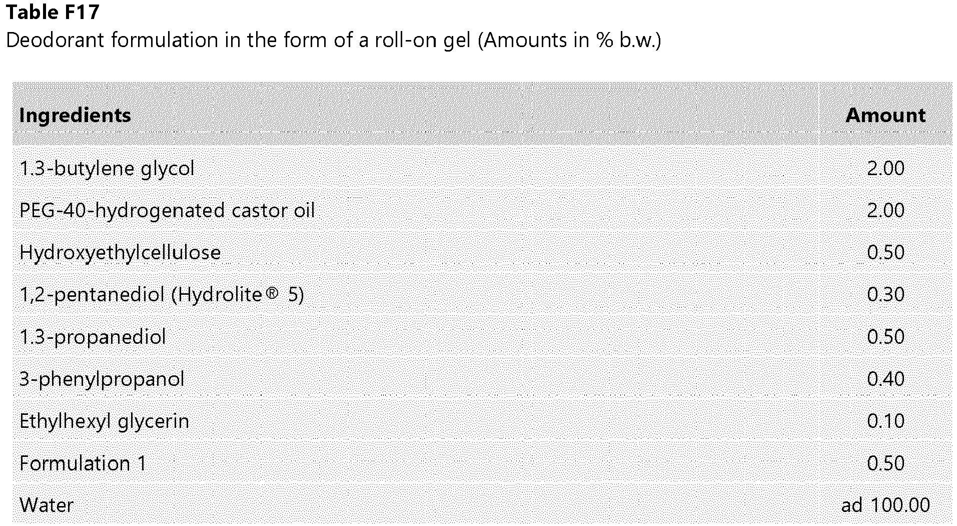

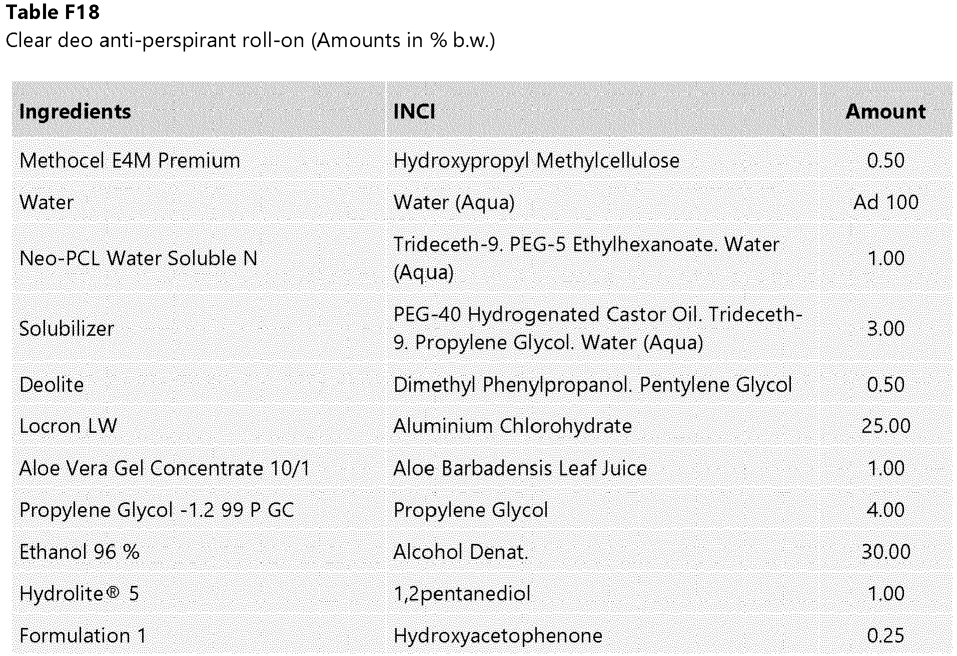

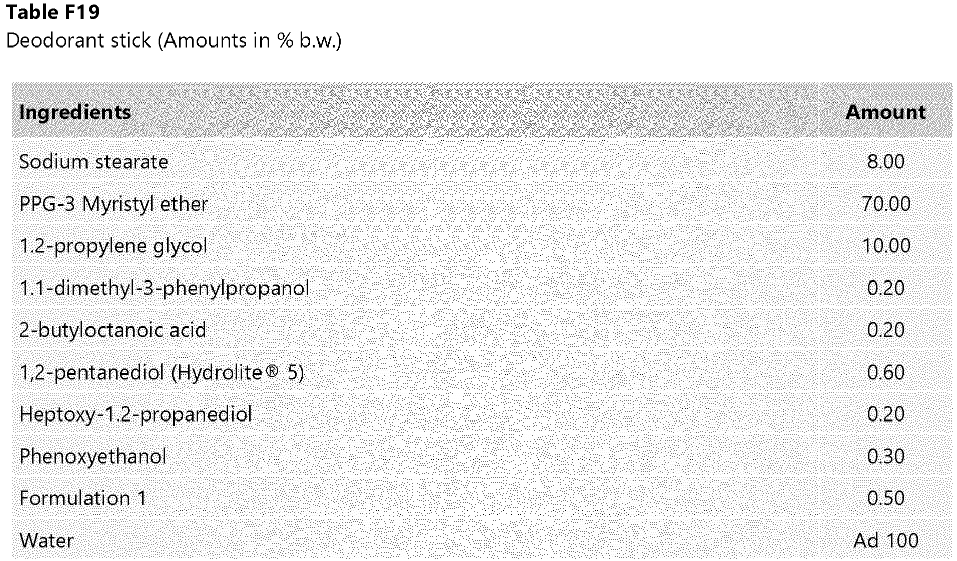

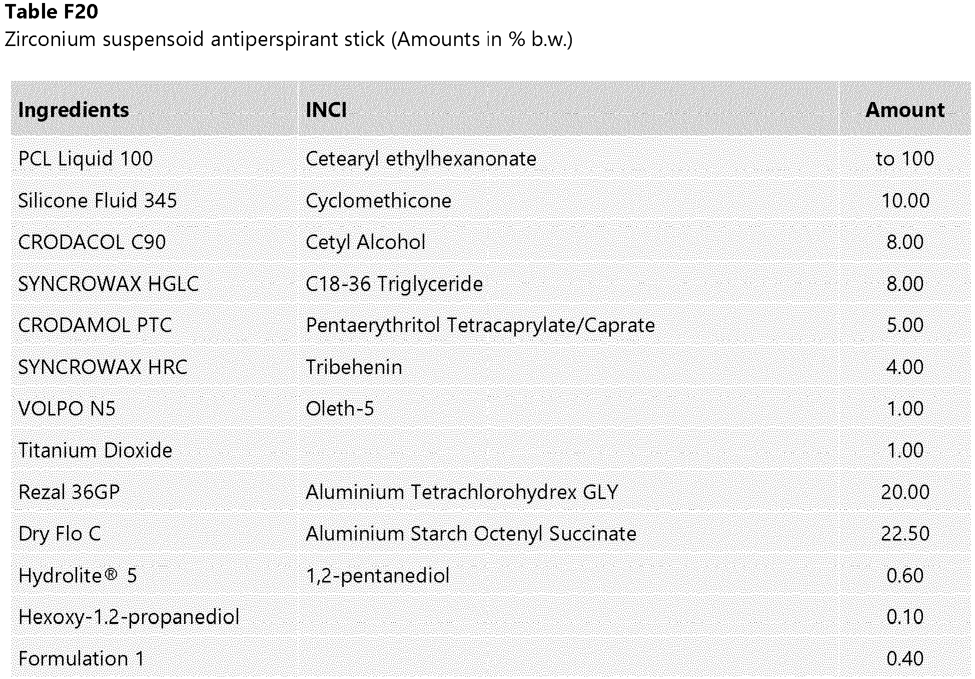

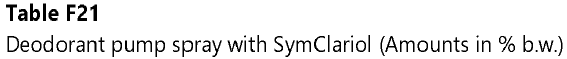

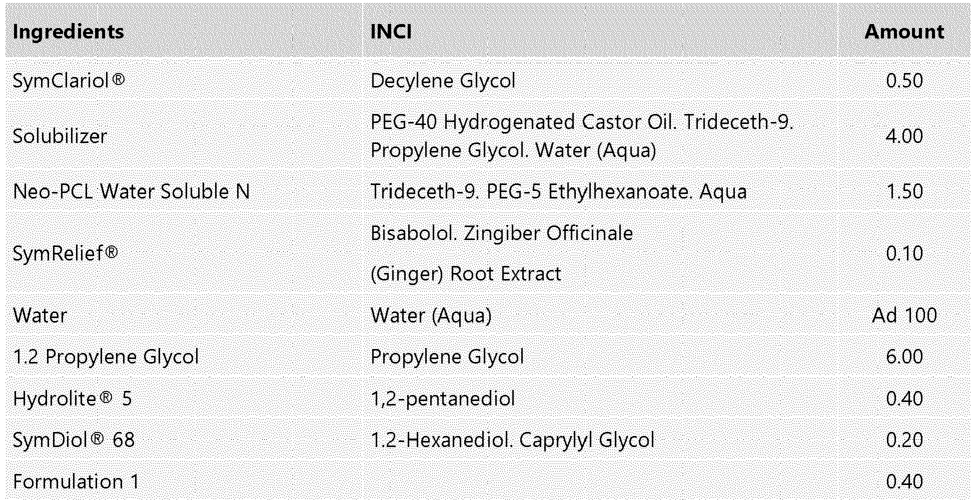

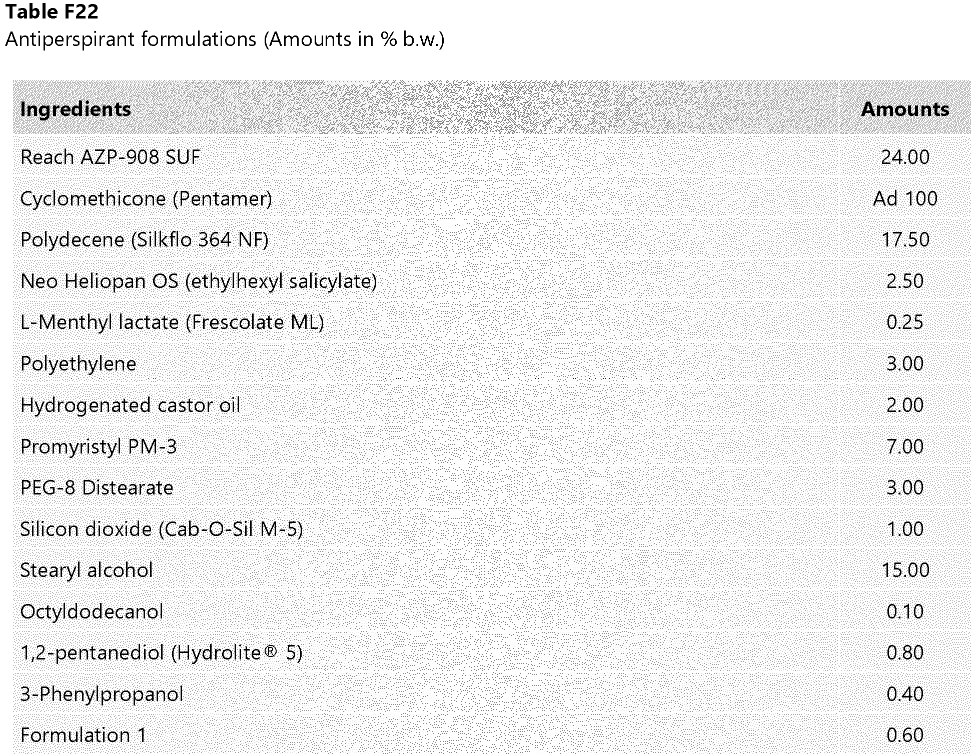

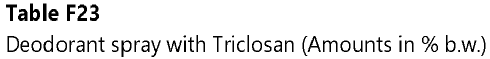

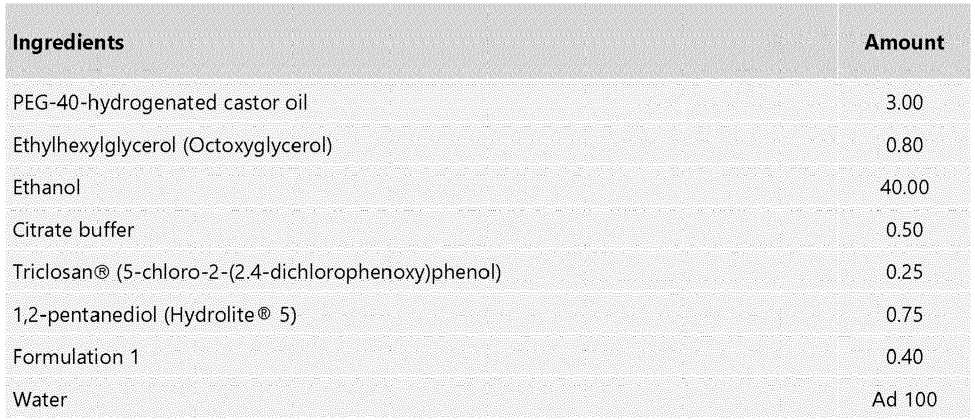

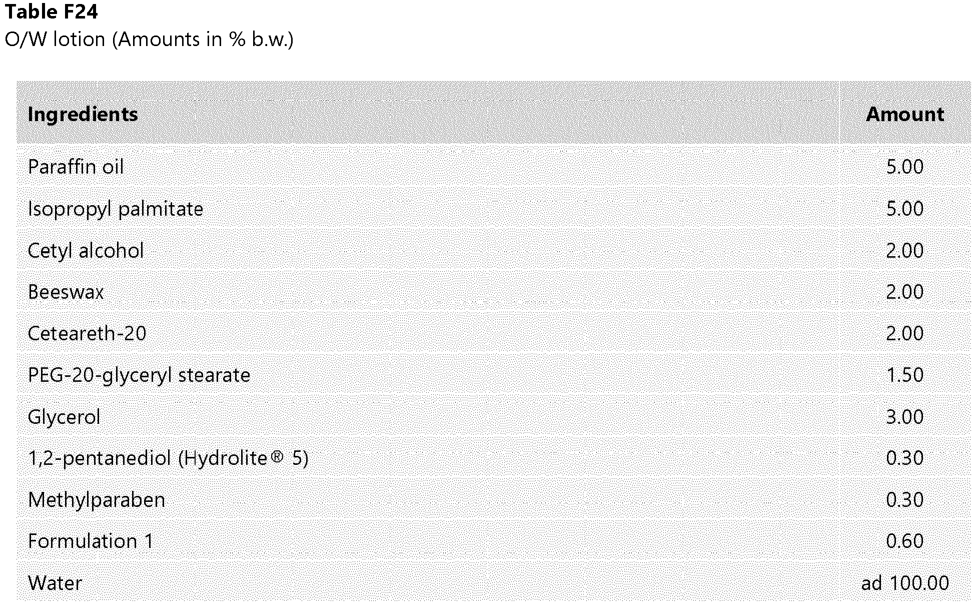

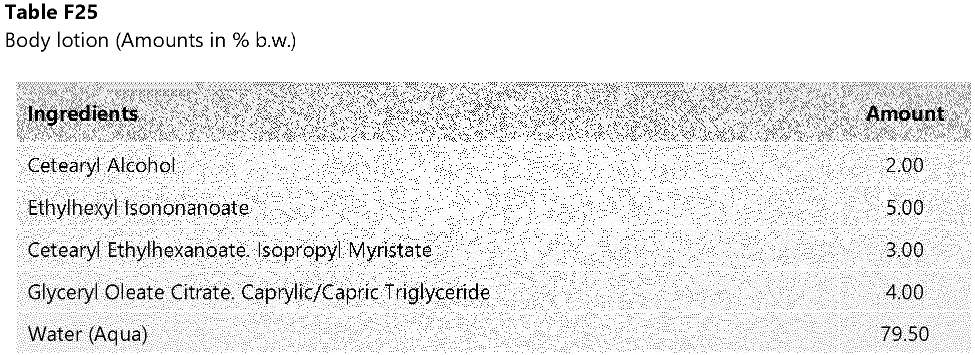

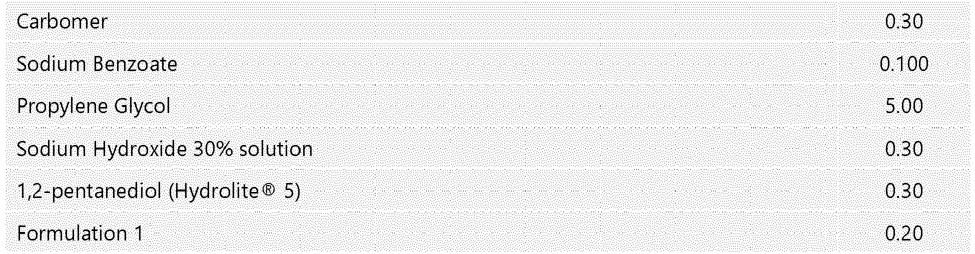

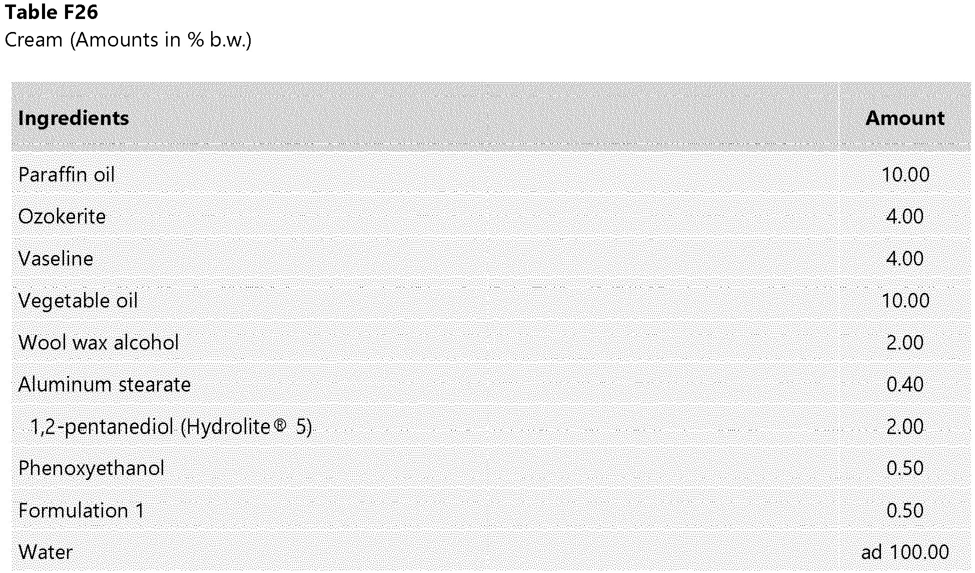

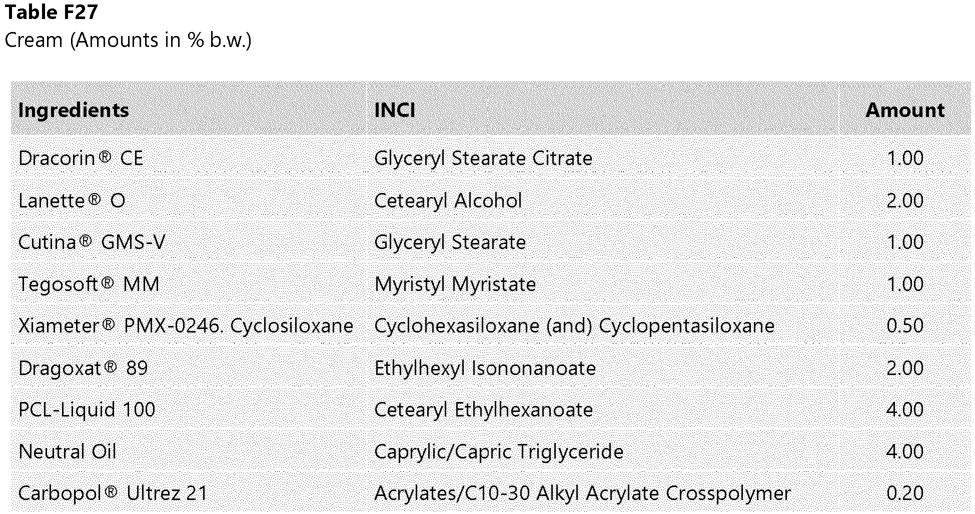

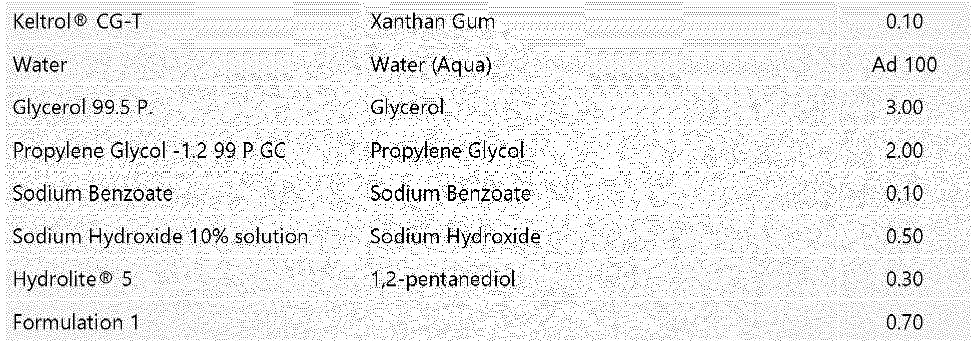

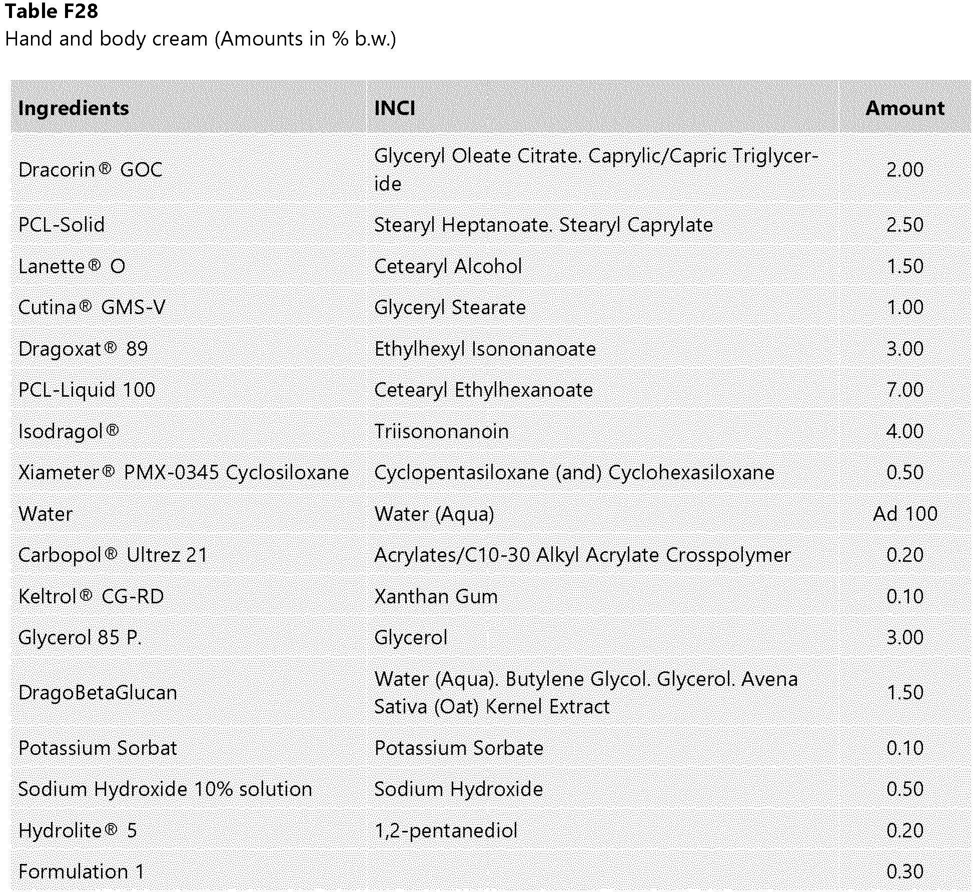

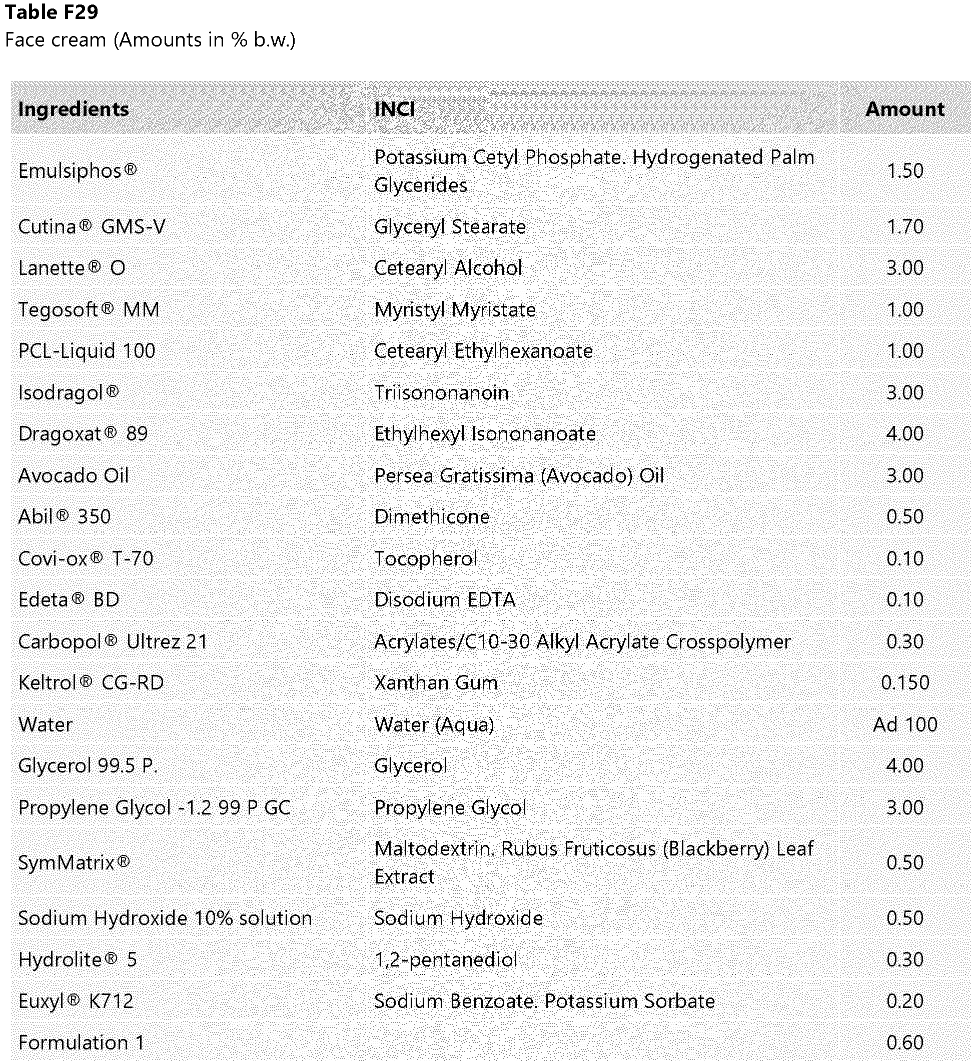

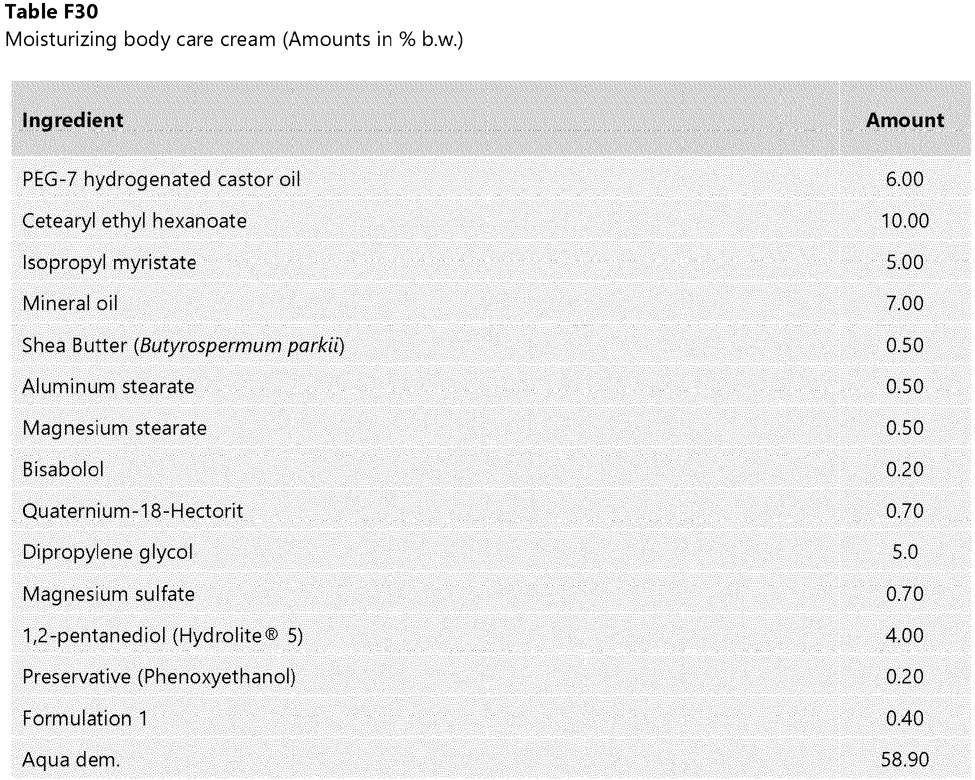

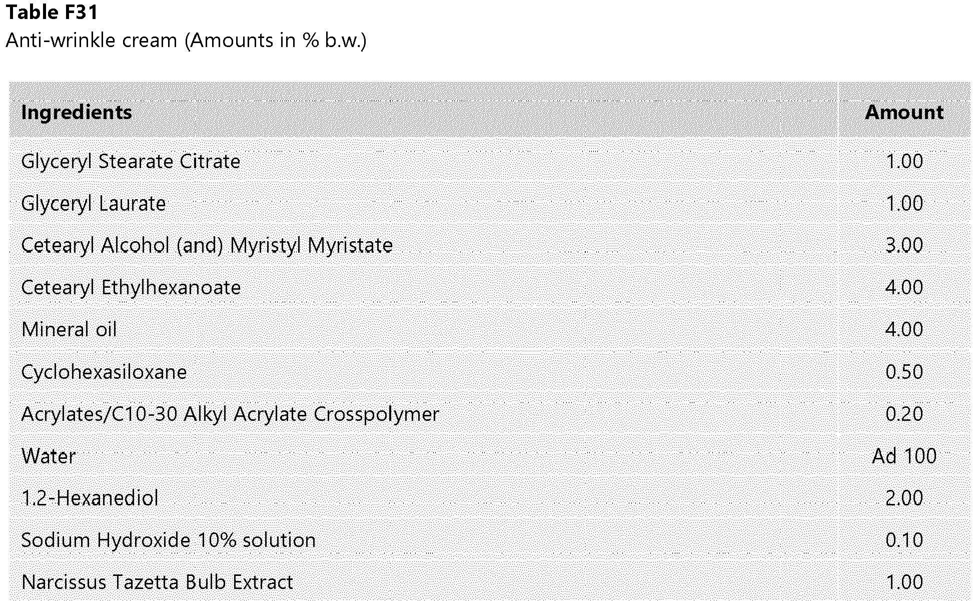

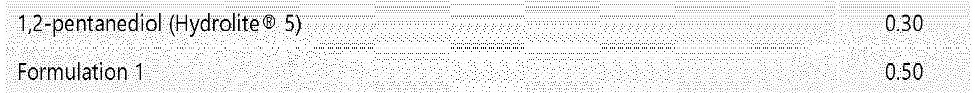

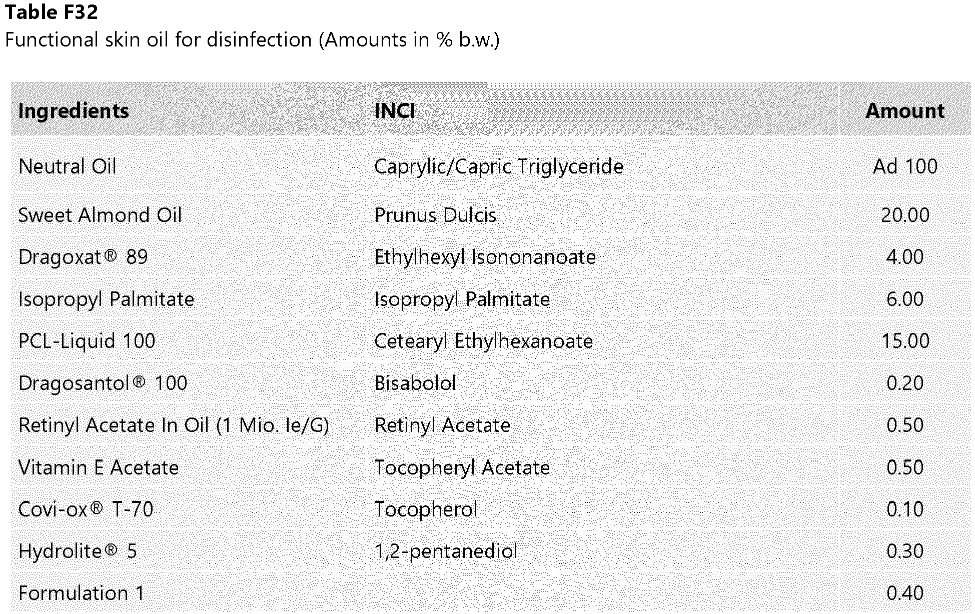

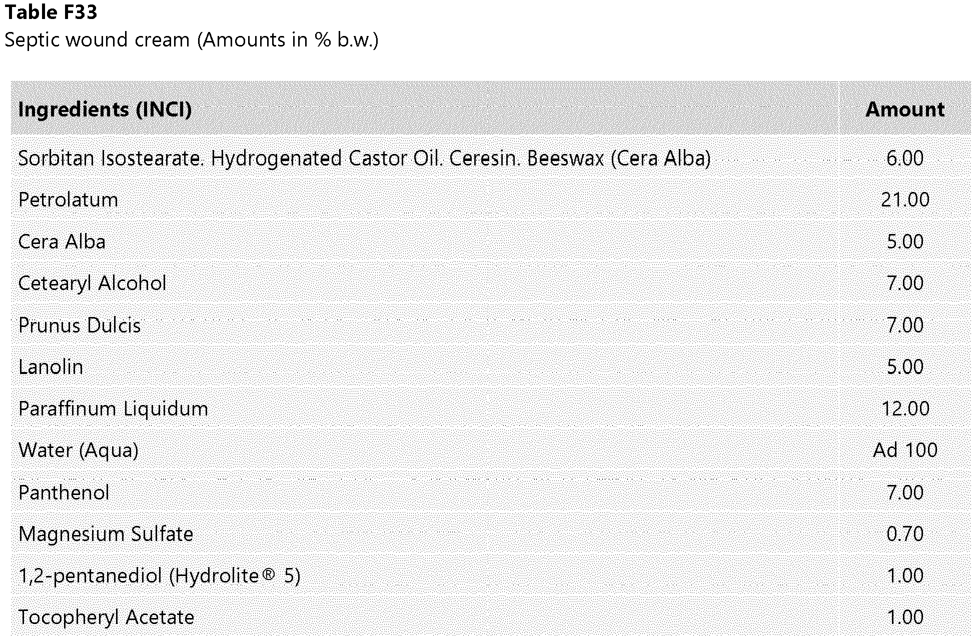

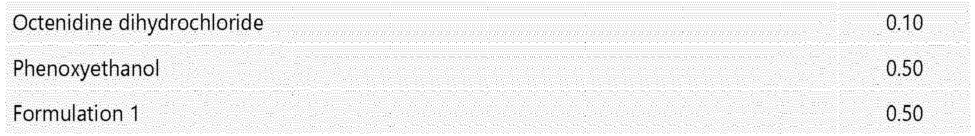

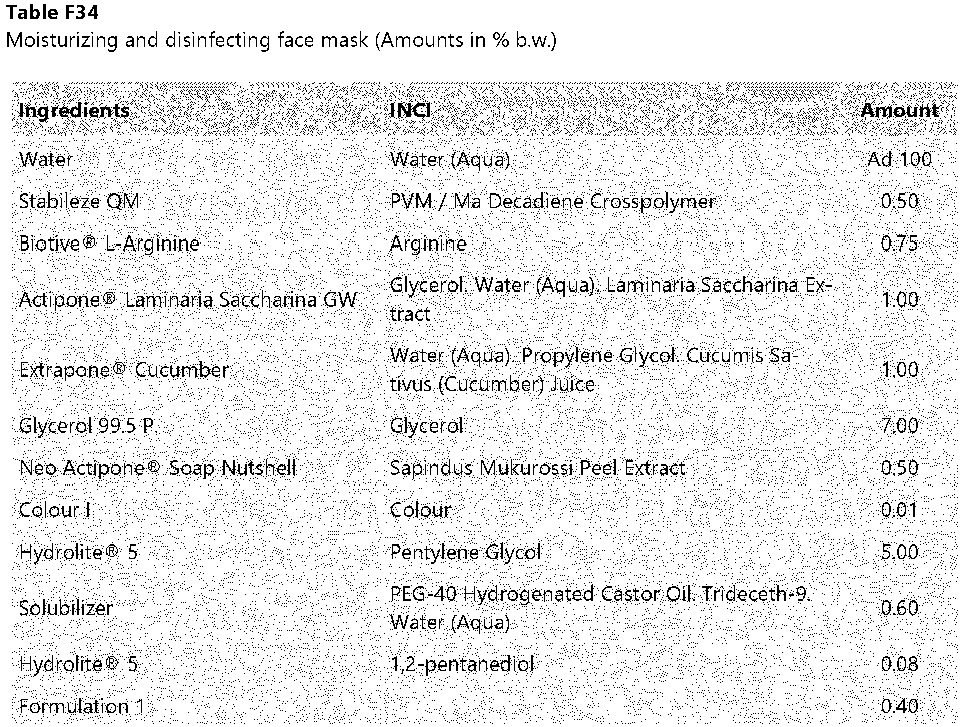

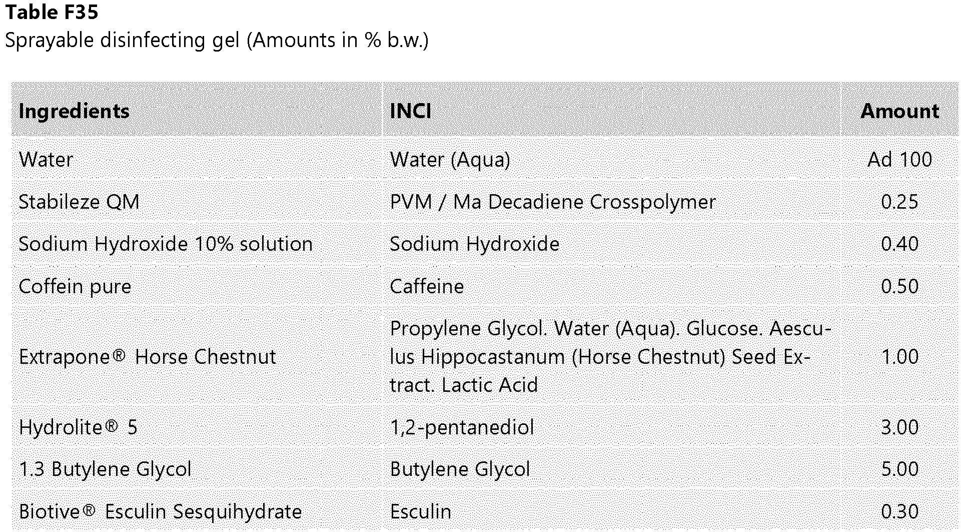

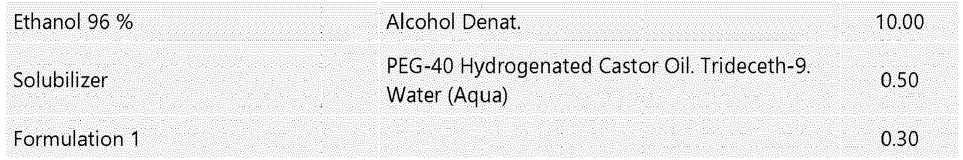

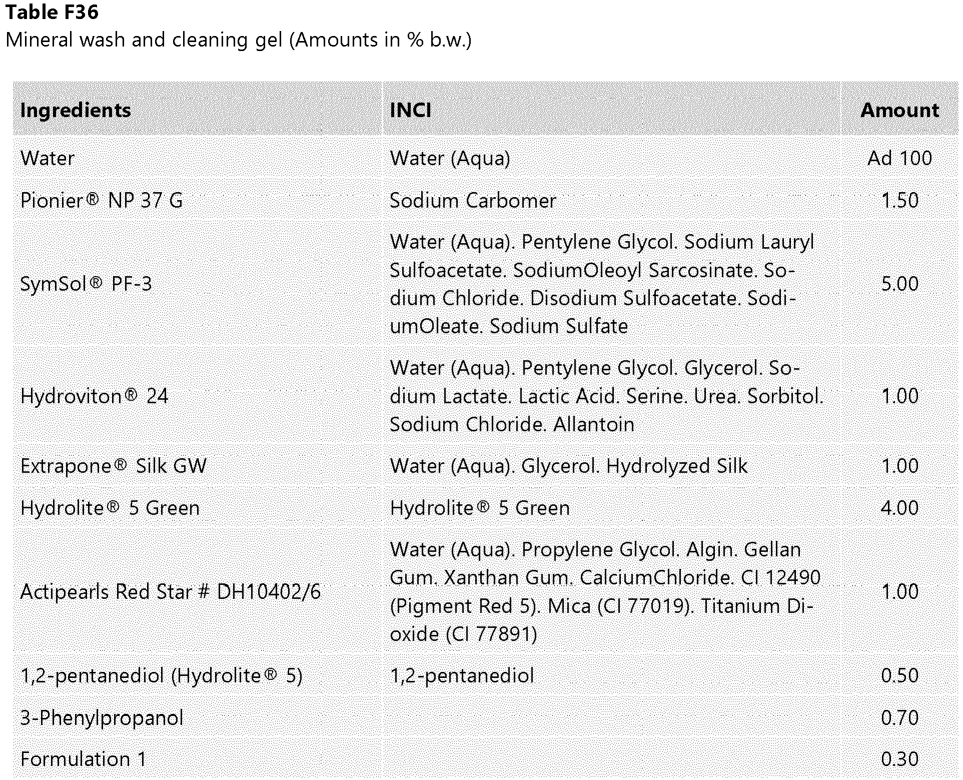

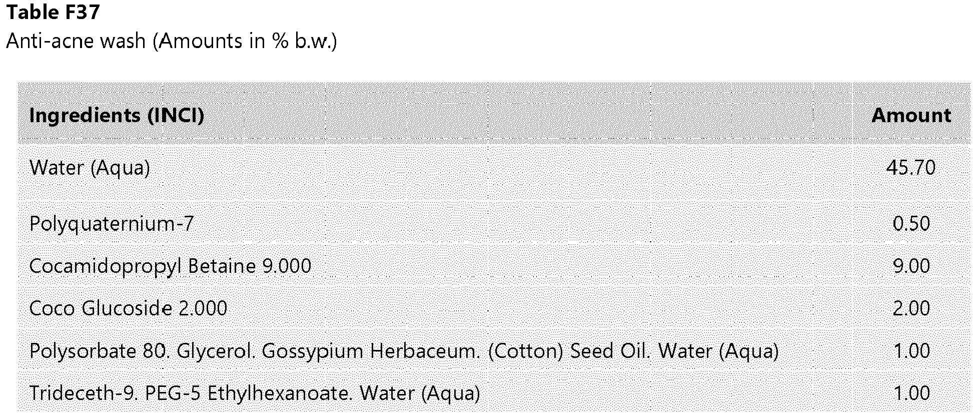

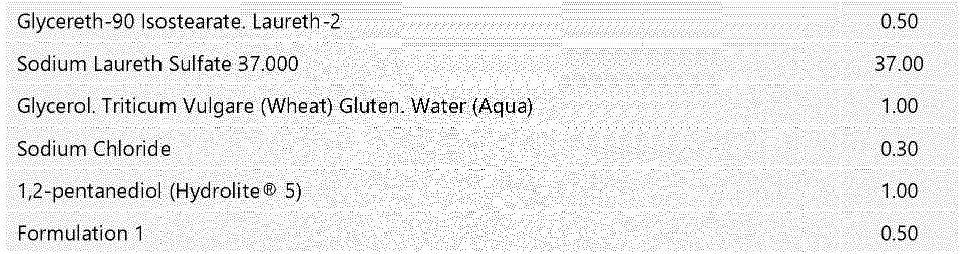

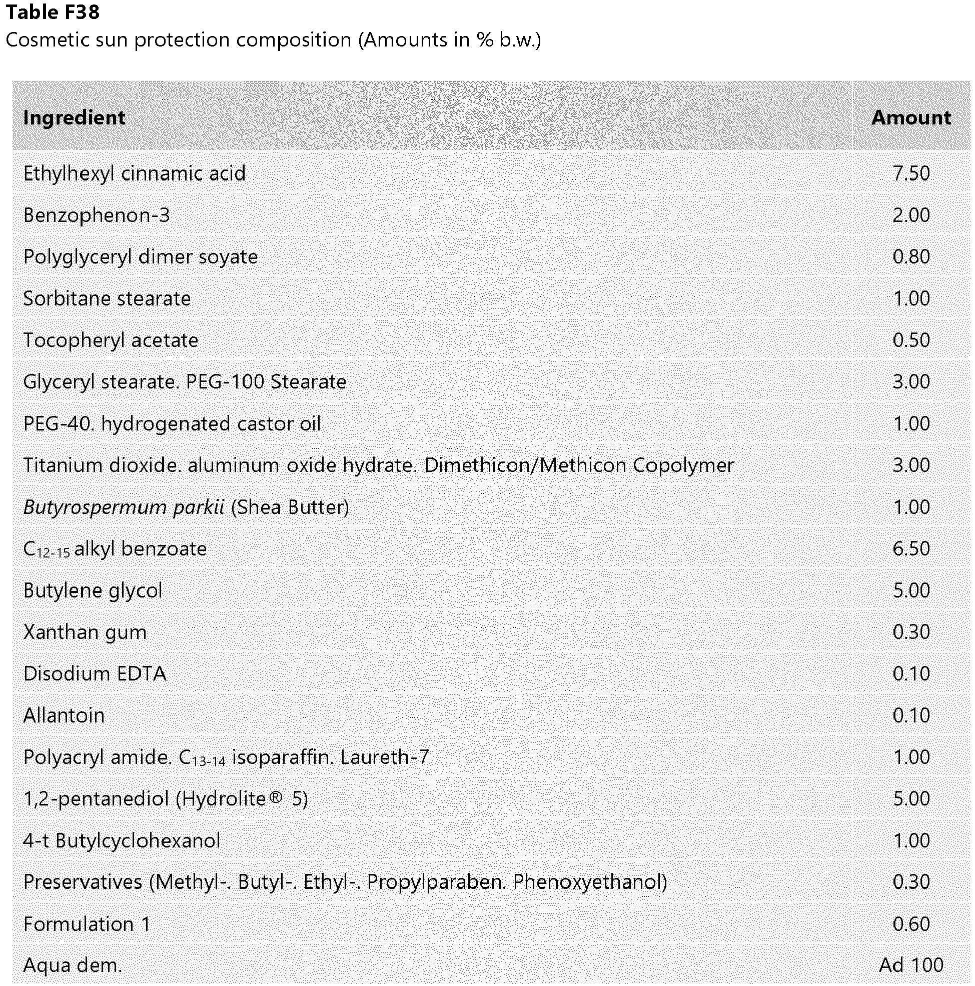

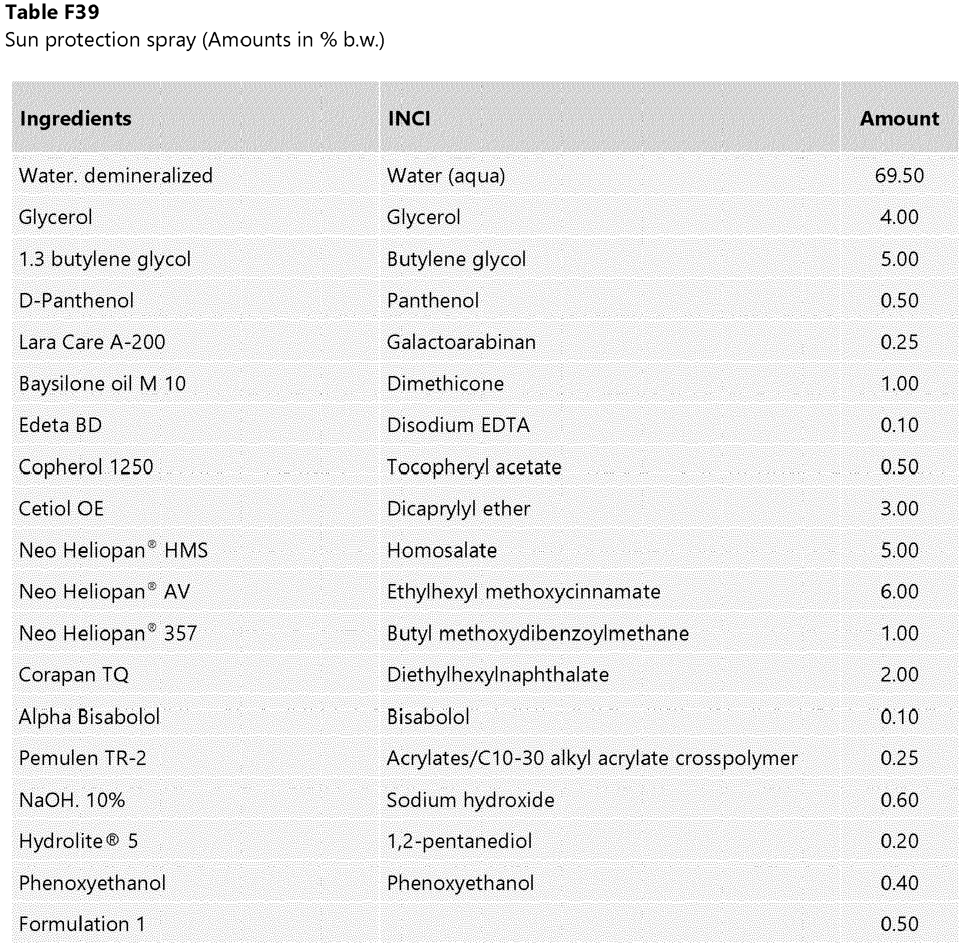

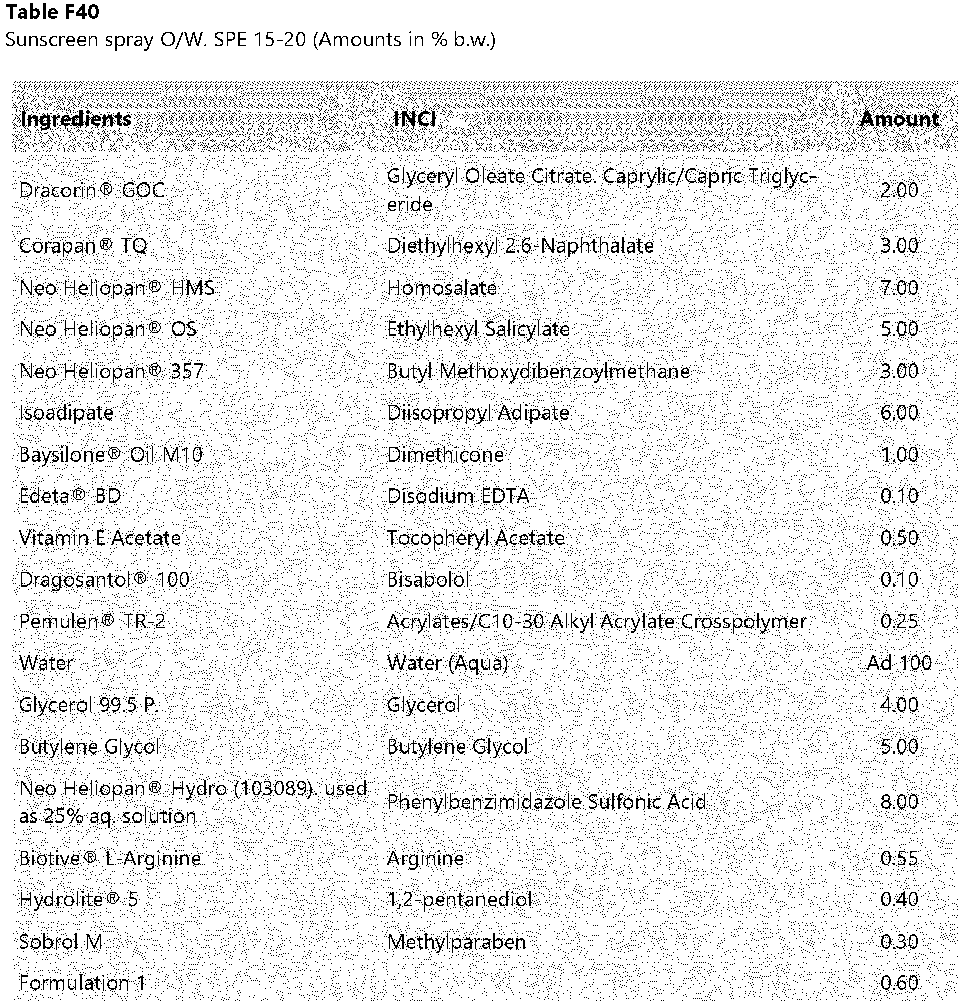

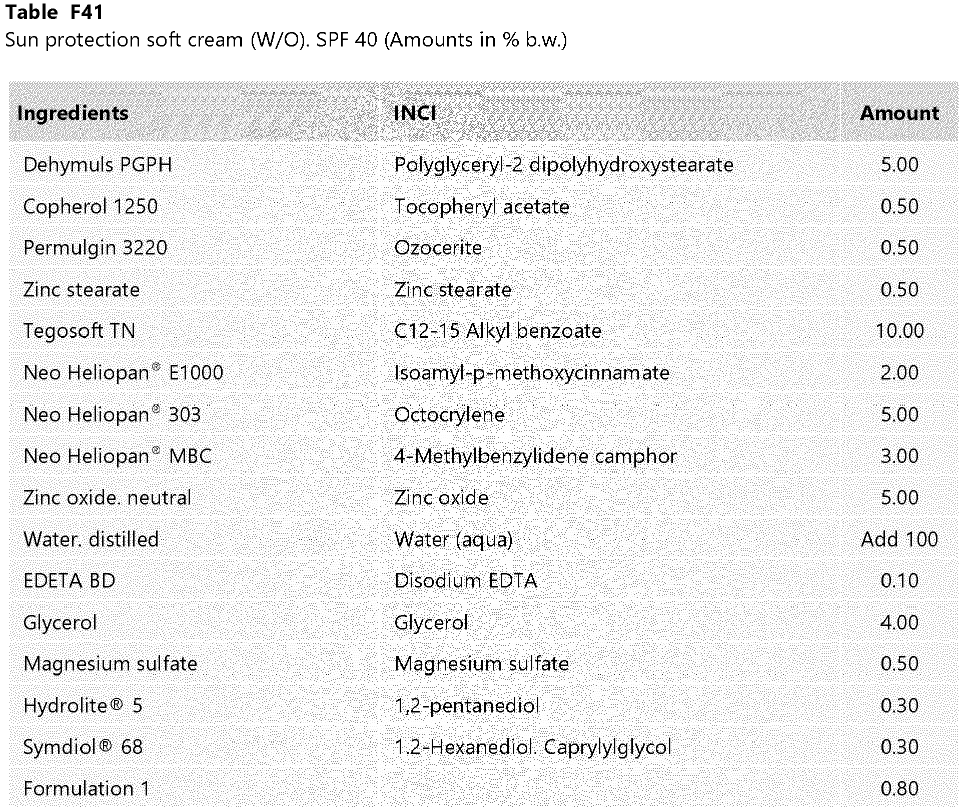

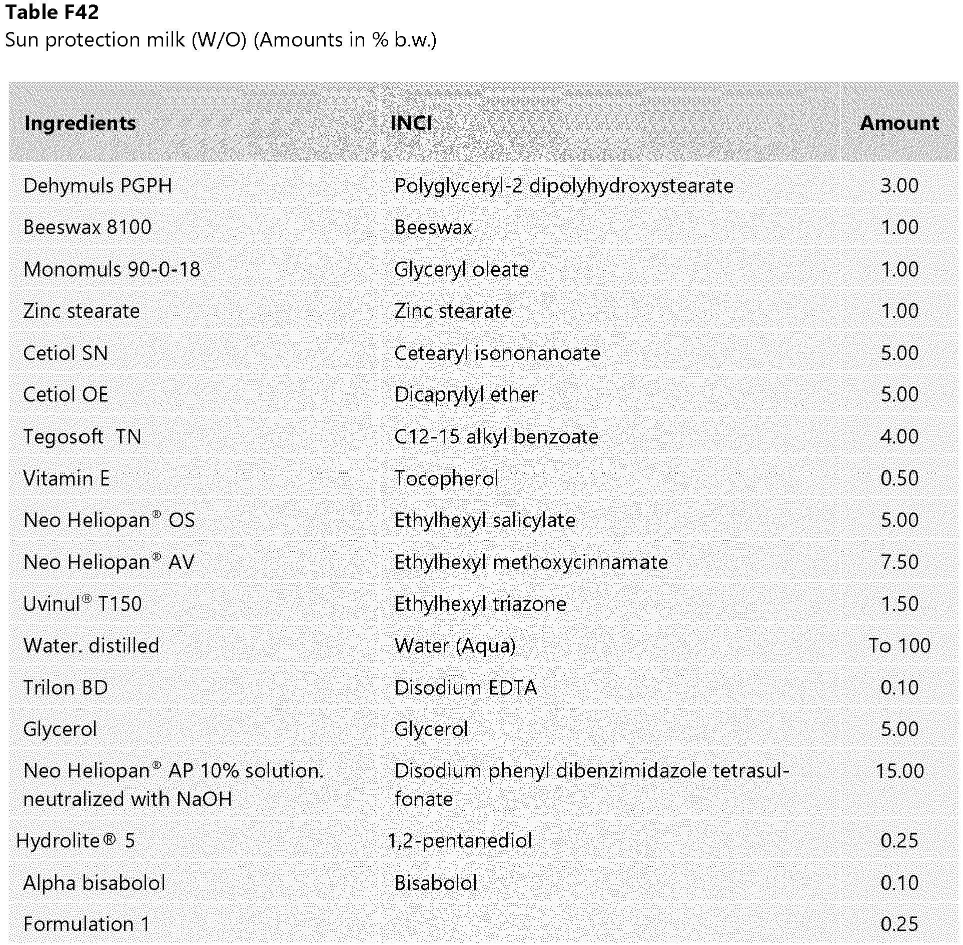

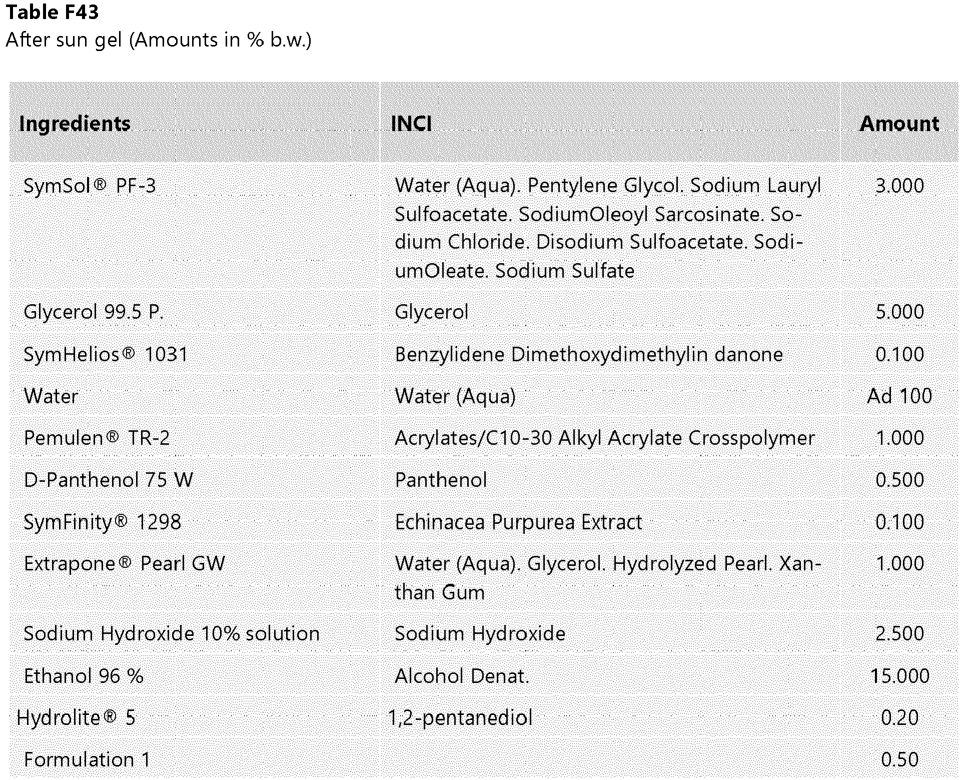

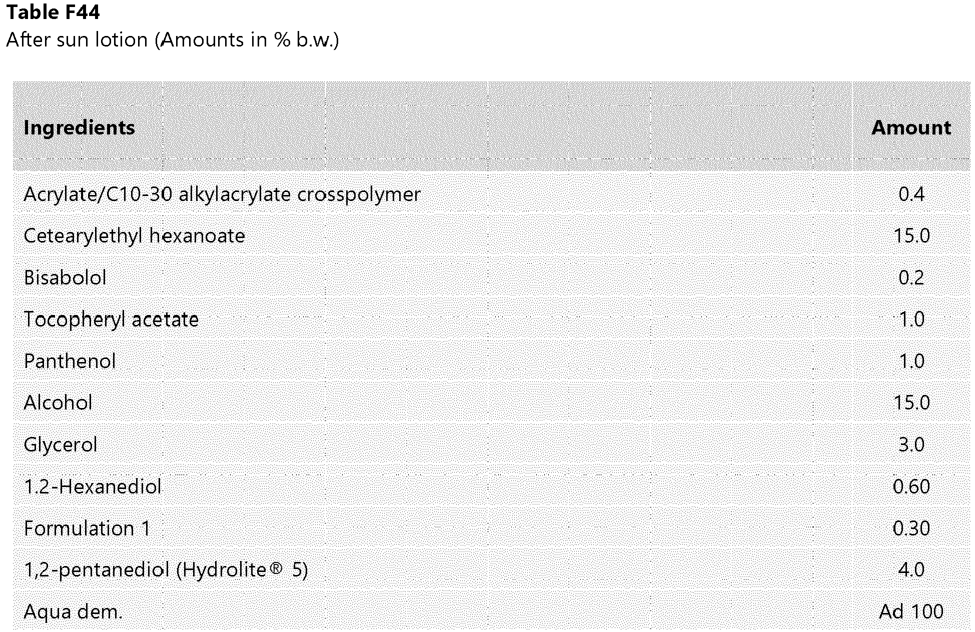

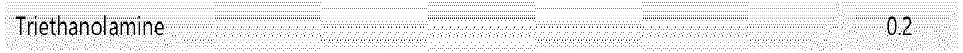

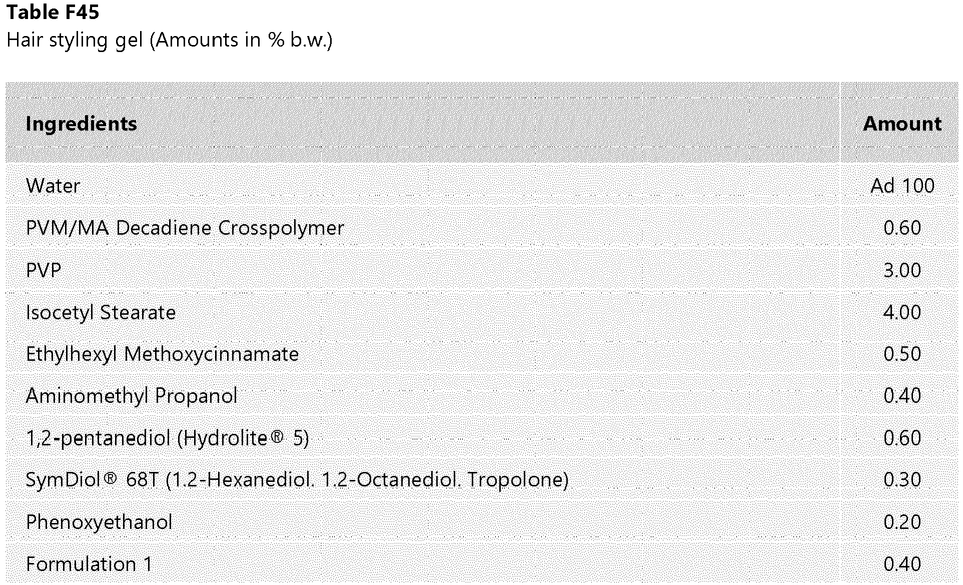

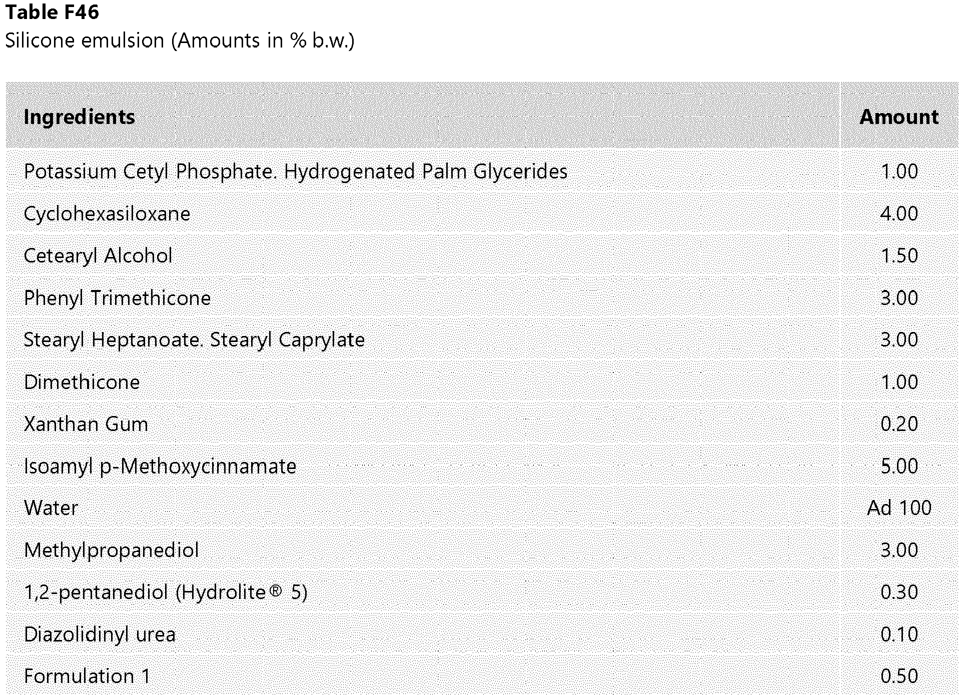

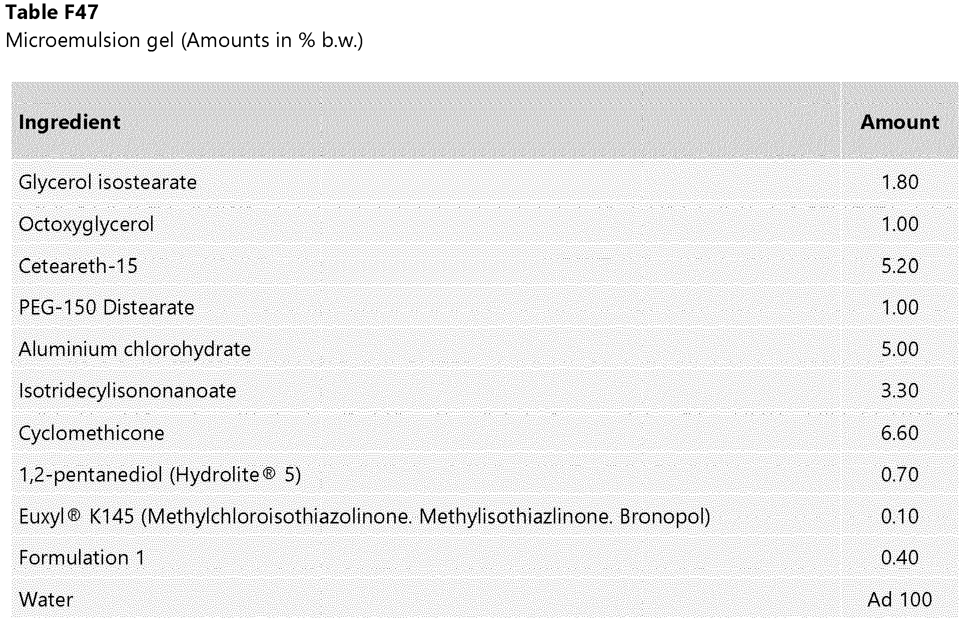

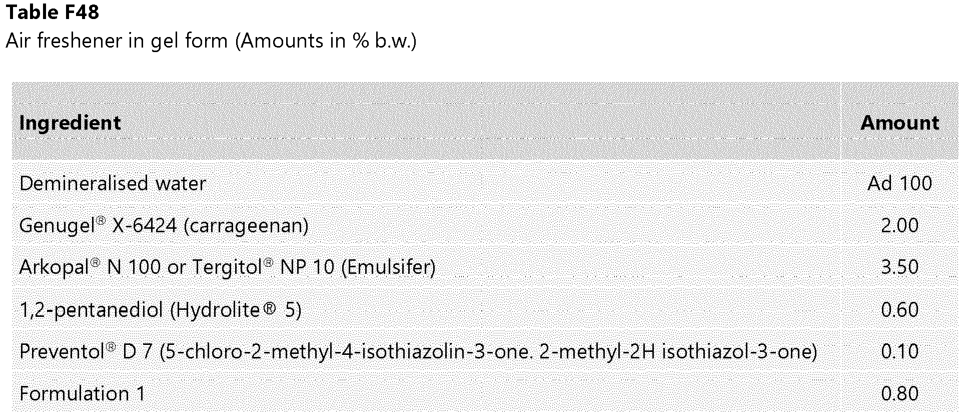

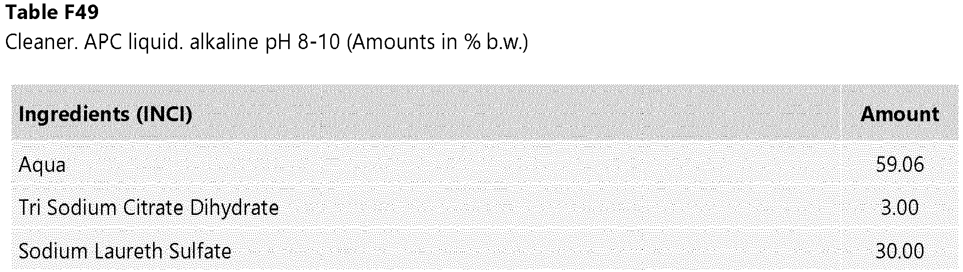

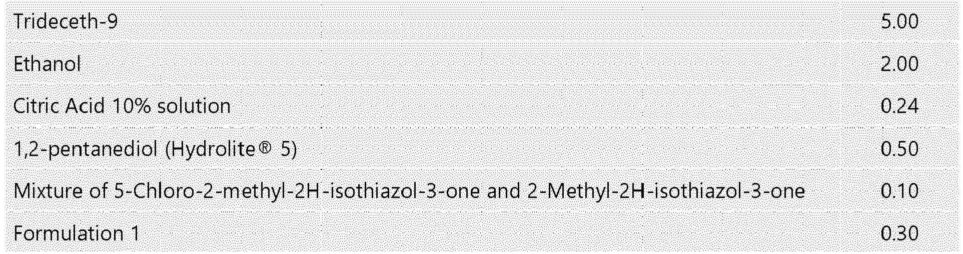

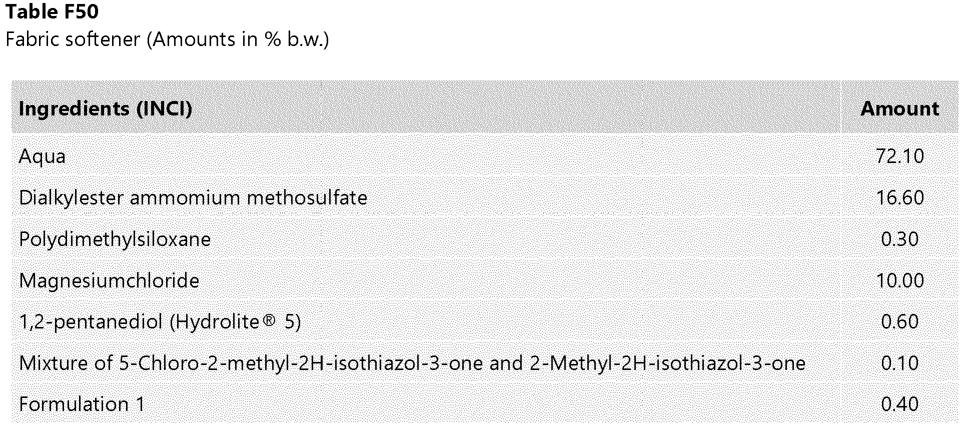

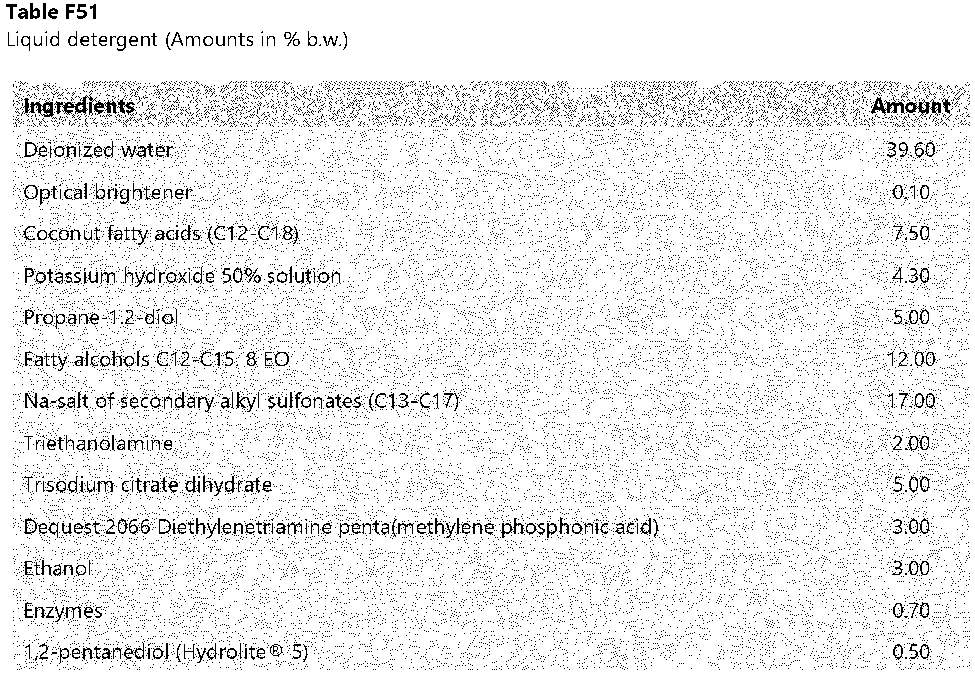

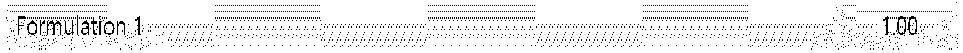

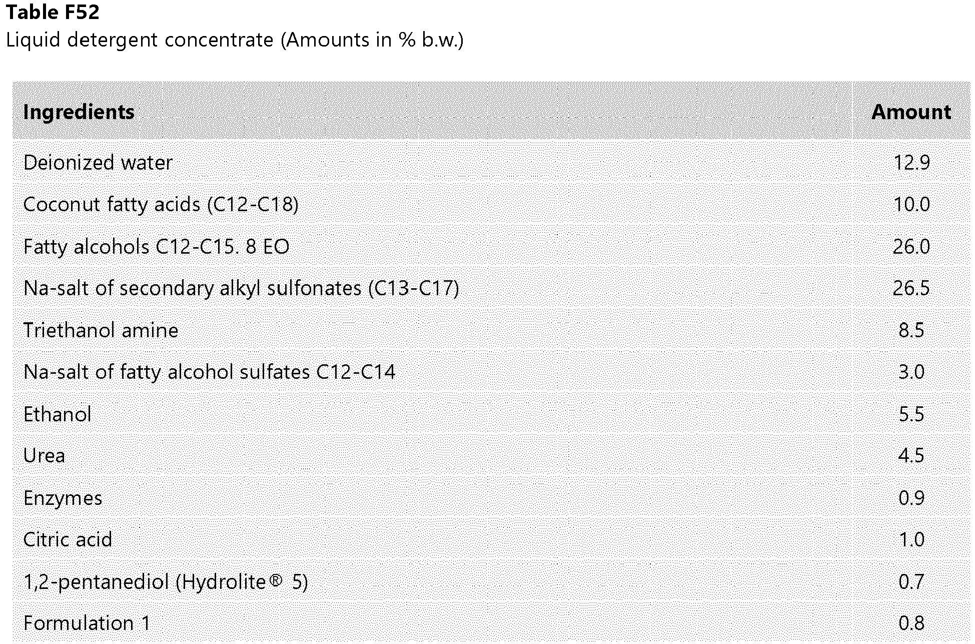

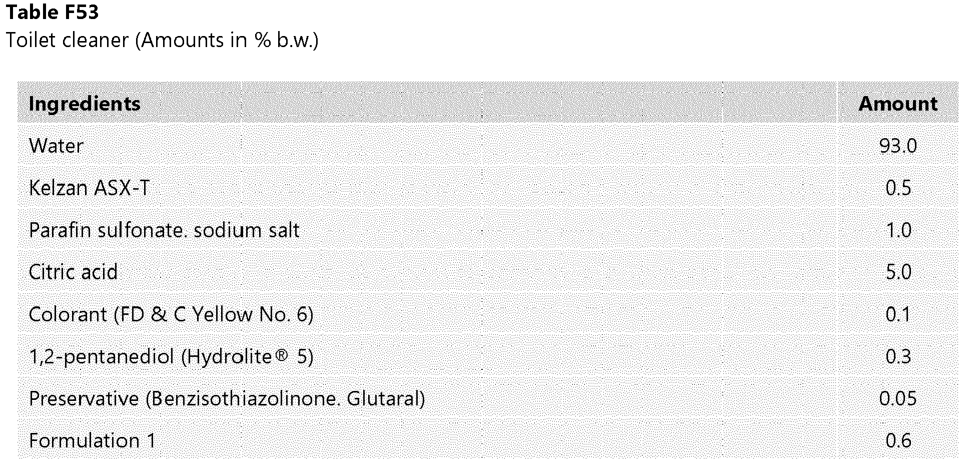

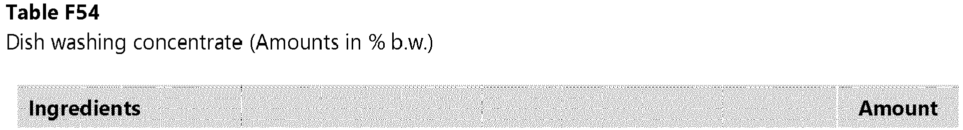

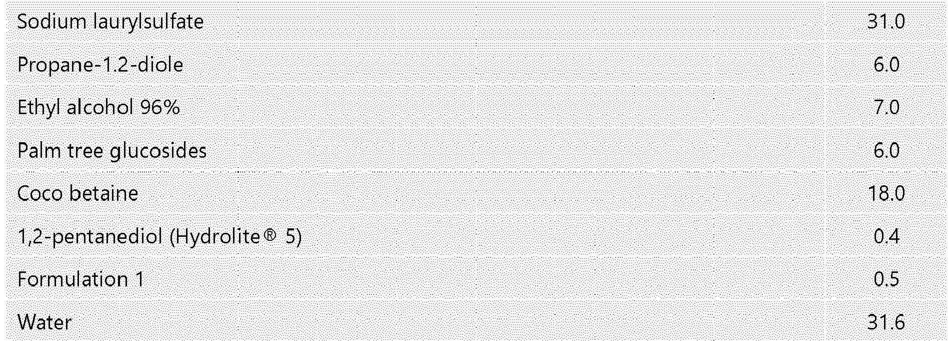

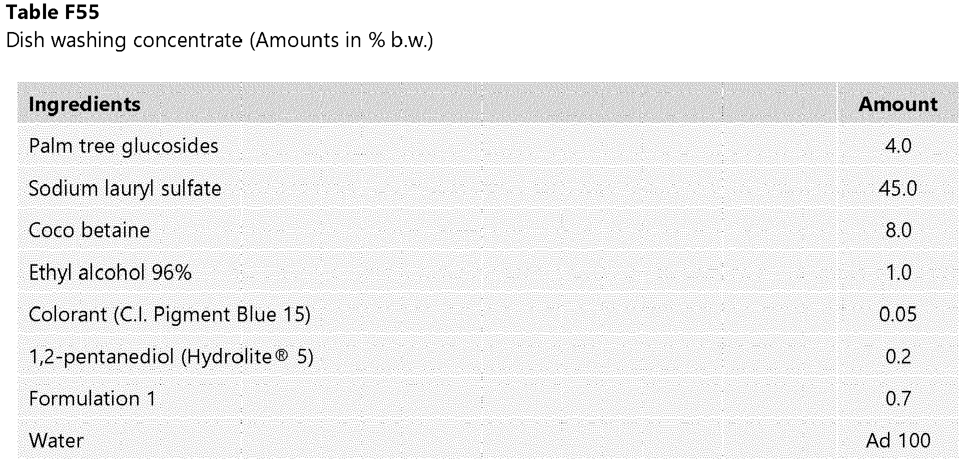

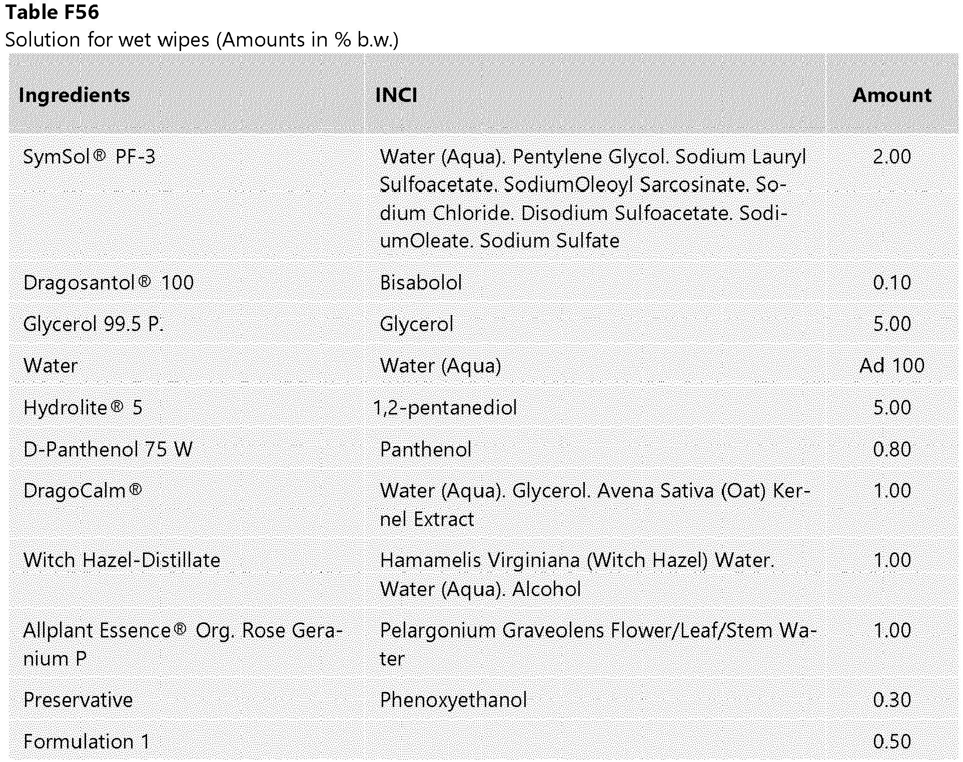

## Claims

1. A preservative composition comprising or consisting of triterpene glycosides, said triterpene glycosides being selected from at least one monodesmosidic hederagenin-3-glycoside according to formula (I) 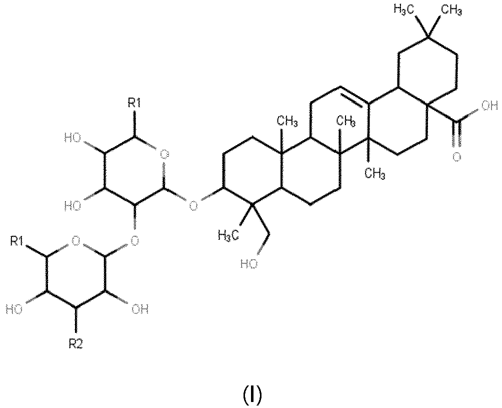 wherein
R1 stands for H, CH3, CH2OH or COOH,
R2 stands for one sugar selected from the group Glu, Gal, Rha, Ara, Xyl, Api, GIcA and mixtures thereof.

2. The composition of Claim 1, wherein said hederagenin glycosides are selected from the group consisting of the following structures A to C:
| **Compound** | **Name** | **Structure** |
|---|---|---|
| A | Sapindoside B [30994-75-3] | 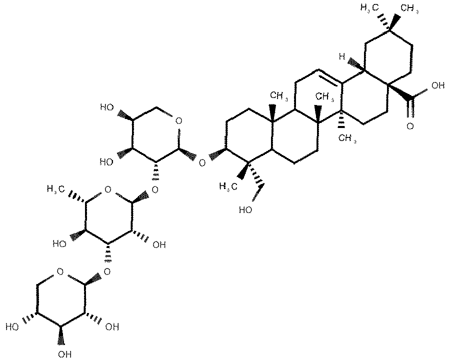 |
| B | Clemontano-side C [80666-65-5] | 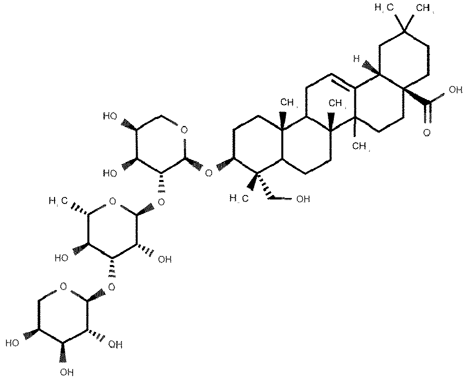 |
| C | Transsylvanoside H [174423-50-8] | 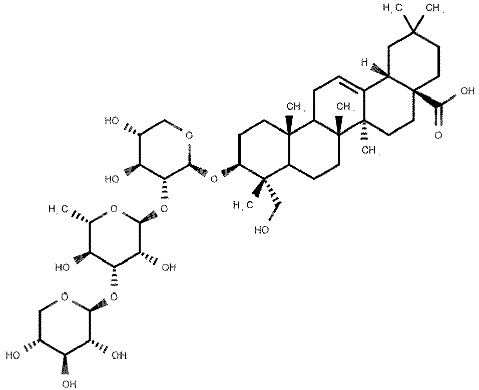 |

3. The composition of Claim 1 comprising said Hederagenin glycosides in amounts ranging from 0.0000001 to 1.0 wt.-percent - calculated on the total composition.

4. The composition of Claim 1 comprising said Hederagenin glycosides in amounts ranging from 0.000001 to 0.1 wt.-percent - calculated on the total composition.

5. The composition of Claim 1 comprising said Hederagenin glycosides in amounts ranging from 0.00001 to 0.01 wt.-percent - calculated on the total composition.

6. The composition of Claim 1, further comprising at least one preservative (component b) different from compounds according to formula (I) (component a).

7. The composition of Claim 6, wherein said additional preservative (component b) is selected from the group consisting of benzoic acid and para-hydroxybenzoic acid, their esters and salts, benzyl benzoate, propionic acid and its salts, salicylic acid and its salts, 2,4-hexadienoic acid (sorbic acid) and its salts, levulinic acid and its salts, anisic acid and its salts, perillic acid and its salts, cinnamic acid and its salts, formaldehyde and paraformaldehyde, 4-hydroxy benzaldehyde, ortho-, meta-, and para-anisic aldehyde, cinnamic aldehyde, cinnamic alcohol, 2-hydroxybiphenyl ether and its salts, 2-zinc-sulfidopyridine N-oxide, inorganic sulfites and bisulfites, sodium iodate, chlorobutanolum, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexahydropy-rimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexamethylene-diguanide) hydrochloride, (Benzyloxymethoxy)-methanol hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, 1,2-dibromo-2,4-dicyanobutane, 2,2'-methylene-bis(6-bromo-4-chlorophenol), bromochlorophene, mixture of 5-chloro-2-methyl-3(2H)-isothiazoli-none, 2-methyl-3(2H)-isothiazolinone and with magnesium chloride and magnesium nitrate, 2-Octyl-2H-isothiazol-3-one, 1,2-benzisothiazol-3(2H)-one, 2-benzyl-4-chlorophenol, 3-(4-Chlorphenoxy)-1,2-propanediol (Chlorphenesin), 2-chloroacetamide, chlorhexidine, chlorhexidine acetate, chlorhexidine gluconate, chlorhexidine hydrochloride, N-alkyl(C₁₂-C₂₂)trimethyl-ammonium bromide and chloride, 4,4-dimethyl-1,3-ox-azolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-(C₈-C₁₈)-dimethyl-benzyl-ammonium chloride, alkyl-(C₈-C₁₈)-dimethyl-benzylammonium bromide, alkyl-(C₈-C₁₈)-dimethyl-benzyl-ammonium saccharinate, benzyl hemiformal, 3-iodo-2-propynyl butylcarbamate, sodium hydroxymethyl-aminoacetate or sodium hydroxymethyl-aminoacetate, imidazolidinylurea, diazolidinylurea, sodium hydroxymethylglycinate, DMDM hydantoin, Tropolone, (Ethylendioxy)dimethanol, 2-Brom-2-(brommethyl)pentandinitril, N-(3-Aminopropyl)-N-dodecylpropan-1,3-diamin, α,α',α"-trimethyl-1,3,5-triazine-1,3,5(2H,4H,6H)-triethanol, pyridine-2-thiol-1-oxide, sodium salt, Tetrahydro-1,3,4,6-tetrakis(hydroxymethyl)imid-azo[4,5-d]imidazol-2,5(1H,3H)-dion, 1,3-bis(hydroxymethyl) -1-(1,3,4-tris(hydroxy-methyl)-2,5-dioxoimidazolidin-4-yl)urea (Diazolidinyl Urea), 1,3-Bis(hydroxy-methyl)-5,5-dimethylimidazolidine-2,4-dione, 3-Acetyl-2-hydroxy-6-methyl-4H-pyran-4-one, cetyl pyridium chloride, caprylhydroxamic acid, sorbohydroxamic acid and their mixtures; 1,3-propanediol, methyl propanediol, 1,2-pentanediol, 1,2-hexanediol, 1,2-octanediol, 1,2-decanediol, 1,5-pentanediol, 1,6-hexanediol, 1,8-octanediol, 1,2-decanediol, ethylhexylglycerin, hexoxy-propan-1,2-diol, heptoxy- propan-1,2-diol, octoxy-propan-1,2-diol, 3-phenoxy-propan-1,2-diol, 3-benzyloxy-propan-1,2-diol, 3-phenylethyloxy-propan-1,2-diol, 3-phenylpropyloxy-propan-1,2-diol, 3-methylbenzyloxy-propan-1,2-diol, sorbitan caprylate, triclosan, climbazole, Octopirox (1-hydroxy-4-methyl-6-(2,4,4-trimethylpentyl)-2(1H)-pyridone, 2-aminoethanol), chitosan, farnesol, 2-butyloctanoic acid, 2-Benzylheptan-1-ol, glycerol monolaurate, bis(2-pyridylthio)zinc 1,1'-dioxide, N,N'-(decane-1,10-diyldipyridin-1-yl-4-ylidene)-dioctan-1-amine dihydrochloride (octenidine dihydrochloride), thymol, eugenol, benzyl alcohol, 2-phenyethyl alcohol, 3-phenyl propanol, 2-phenoxyethanol, 1-phenoxy-propan-2-ol, 3-phenoxypropanol, benzyloxymethanol, 4-hydroxyacetophenone and mixtures thereof.

8. The composition of Claim 6, comprising components a:b in ratios by weight ranging from about 0.1:99.9 to 99:1.

9. The composition of Claim 1 being active against at least one of the following microorganisms:
• *Actinomyces viscosus*
• *Alicyclobacillus acidoterrestris*
• *Alternaria alternata*
• *Aspergillus brasiliensis*
• *Bacillus cereus*
• *Bacillus licheniformis*
• *Botrytis cinerea*
• *Brettanomyces naardenensis*
• *Byssochlamys fulva*
• *Campylobacter jejuni*
• *Candida albicans*
• *Candida parapsilosis*
• *Cladosporium cladosporioides*
• *Clostridium perfringens*
• *Clostridium sporogenes*
• *Corynebacterium xerosis*
• *Cutibacterium acnes*
• *Enterobacter cloacae*
• *Enterococcus faecalis*
• *Fusobacterium nucleatum subsp. vincentii*
• *Gluconacetobacter liquefaciens*
• *Lactobacillus paracasei ssp. paracasei*
• *Lactobacillus plantarum ssp. plantarum*
• *Leuconostoc lactis*
• *Leuconostoc mesenteroides ssp. mesenteroides*
• *Listeria monocytogenes*
• *Malassezia furfur*
• *Pasteurella multocida ssp. multocida*
• *Penicillium expansum*
• *Phanerochaete chrysosporium*
• *Porphyromonas gingivalis*
• *Pseudomonas aeruginosa*
• *Pseudomonas fluorescens*
• *Saccharomyces cerevisiae*
• *Salmonella enterica ssp. enterica Ser. typhimurium*
• *Staphylococcus aureus*
• *Streptococcus mutans*
• *Yarrowia lipolytica*
• *Zygosaccharomyces bailii*
• *Zygosaccharomyces rouxii.*

10. A method for obtaining Hederagenin glycosides according to Claim 1 encompassing following steps:
(d) providing a source of monodesmosidic hederagenin-3-glycosides;
(e) providing a solvent selected from the group consisting of water, C1 to C4 alcohols, acetone, methyl-ethyl-ketone or mixtures thereof;
(f) bringing the Hederagenin glycosides according to step (a) in contact with said solvent or solvent mixture according to step (b), optionally
(c1) adjusting the pH value of the mixture thus obtained to about 10 to about 14 and/or
(c2) heating the mixture thus obtained to a temperature of from about 40°C to about 90°C and/or
(c3) extracting the hederagenin glycosides from the mixture thus obtained with n-butanol or by adsorption on a resin followed by elution with methanol to get an extract enriched with hederagenin glycosides
to obtain an extract,
wherein said source of monodesmosidic hederagenin-3-glycosides is a plant material derived from plants belonging to the genera of
*VIII. Sapindus,*
*IX. Aesculus,*
*X. Hedera,*
*XI. Blighia,*
*XII. Dipsacus,*
*XIII. Aralia* and/or
*XIV. Anemone*
or mixtures thereof.

11. A method for improving antimicrobial stability of a cosmetic, detergent or food preparation encompassing the following steps:
(a) providing a preparation with low or no antimicrobial stability and
(b) adding a working amount of the preservative composition of Claim 1 or Claim 6.

12. A cosmetic preparation comprising the preservative composition of Claim 1 or Claim 6.

13. A detergent composition comprising the preservative composition of Claim 1 or Claim 6.

14. A food preparation comprising the preservative composition of Claim 1 or Claim 6.

15. The use of the preservative composition of Claim 1 or Claim 6 for antimicrobial stabilization of cosmetic, detergent or food preparations.
